# EUROPÄISCHE PATENTSCHRIFT

(11) **EP 1 135 479 B1**
(45) Veröffentlichungstag und Bekanntmachung des Hinweises auf die Patenterteilung: **18.12.2002**
(21) Anmeldenummer: 99948662.4
(22) Anmeldetag: 23.07.1999
(51) Int. Cl.: C12N 15/10

(54) **FORMULIERUNGEN UND VERFAHREN ZUR ISOLIERUNG VON NUKLEINSÄUREN AUS BELIEBIGEN KOMPLEXEN AUSGANGSMATERIALIEN UND NACHFOLGENDE KOMPLEXE GENANALYTIK**
FORMULATIONS AND METHODS FOR ISOLATING NUCLEIC ACIDS FROM ANY COMPLEX STARTING MATERIAL AND SUBSEQUENT COMPLEX GENETIC ANALYSIS
FORMULATION ET TECHNIQUE PERMETTANT D'ISOLER DES ACIDES NUCLEIQUES A PARTIR D'UN MATERIEL DE DEPART COMPLEXE, ET ANALYSE GENIQUE COMPLEXE LEUR FAISANT SUITE

(30) Priorität: 04.12.1998 DE 19856064
(43) Veröffentlichungstag der Anmeldung: 26.09.2001
(73) Patentinhaber: Invitek Gesellschaft für Biotechnik & Biodesign mbH., 13125 Berlin (DE)
(72) Erfinder: HILLEBRAND, Timo, D-12619 Berlin (DE); BENDZKO, Peter, D-12623 Berlin (DE)
(74) Vertreter: Baumbach, Friedrich, Dr.
(86) Internationale Anmeldenummer: DE9902248
(87) Internationale Veröffentlichungsnummer: WO00034463

(56) Entgegenhaltungen:
- EP-A- 0 376 080
- EP-A- 0 442 026
- EP-A- 0 512 767
- EP-A- 0 648 776
- WO-A-95/34569
- WO-A-96/18905
- US-A- 5 693 785

## Beschreibung

Gegenstand der Erfindung sind Formulierungen ohne chaotrope Bestandteile zur Isolierung von Nukleinsäuren unter Bindung an eine feste Phase, insbesondere von DNA aus beliebigen komplexen Ausgangsmaterialien und Mengen enthaltend ein Lyse/Bindungspuffersystem, das mindestens eine antichaotrope Salzkomponente aufweist. eine feste Phase und an sich bekannte Wasch- und Elutionspuffer. Das Lyse/Bindungspuffersystem kann als wässerige Lösung vorliegen oder als feste Formulierung in einsatzfertigen Reaktionsgefäßen. Als feste Phase können alle Trägermaterialien fungieren, die zur Isolierung mittels chaotroper Reagentien Anwendung finden, vorzugsweise Glafaservliese, Glasmembranen, Siliciumträger Keramiken, Zeolithe oder Materialien, die negativ funktionalisierte Oberflächen besitzen oder chemisch modifizierte Oberflächen aufweisen, die in ein negatives Ladungspotential überführt werden können.

Gegenstand der Erfindung ist ferner ein Verfahren zur Isolierung von Nukleinsäuren, insbesondere von DNA, aus beliebigen komplexen Ausgangsmaterialien unter Verwendung der erfindungsgemäßen Formulierungen, das durch Lyse des Ausgangsmaterial, Bindung der Nukleinsäuren an ein Trägermaterial, Waschung der am Träger gebundenen Nukleinsäuren und Elution der Nukleinsäuren gekennzeichnet ist, wobei ggf. die nachfolgende Amplifikation von ausgewählten Sequenzabschnitten und ggf. eine nachfolgende Analyse der vervielfältigten Genabschnitte in ein und der selben Reaktionskavität durchgeführt werden kann. Die Anwendungsgebiete der Verfahren sind alle mit DNA-Isolierungen sich beschäftigenden Laboratorien, wie forensische Medizin, Lebensmitteldiagnostik, medizinische Diagnostik, Molekularbiologie, Biochemie, Gentechnik und alle anderen angrenzenden Gebiete.

Unter klassischen Bedingungen erfolgt die Isolierung von DNA aus Zellen und Geweben dadurch, daß die Nukleinsäuren enthaltenden Ausgangsmaterialien unter stark denaturierenden und reduzierenden Bedingungen, teilweise auch unter Verwendung von proteinabbauenden Enzymen aufgeschlossen, die austretenden Nukleinsäurefraktionen über Phenol-/Chloroform-Extraktionsschritte gereinigt und die Nukleinsäuren mittels Dialyse oder Ethanolpräzipitation aus der wäßrigen Phase gewonnen werden (Sambrook, J., Fritsch, E.F. und Maniatis, T., 1989, CSH, "Molecular Cloning").

Diese "klassischen Verfahren" zur Isolierung von Nukleinsäuren aus Zellen und besonders aus Geweben sind sehr zeitaufwendig (teilweise länger als 48 h ), erfordern einen erheblichen apparativen Aufwand und sind darüber hinaus auch nicht unter Feldbedingungen realisierbar. Außerdem sind solche Methoden auf Grund der verwendeten Chemikalien wie Phenol und Chloroform in einem nicht geringen Maße gesundheitsgefährdend.

Verschiedene alternative Verfahren zur Isolierung von Nukleinsäuren aus unterschiedlichen biologischen Ausgangsmaterialien ermöglichen die aufwendige und gesundheitsschädigende Phenol-/Chloroform-Extraktion von Nukleinsäuren zu umgehen sowie eine Reduzierung der zeitlichen Aufwendungen zu erreichen.

Alle diese Verfahren basieren auf einer von Vogelstein und Gillespie (Proc. Natl. Acad. Sci. USA, 1979, 76, 615-619) entwickelten und erstmals beschriebenen Methode zur präparativen und analytischen Reinigung von DNA-Fragmenten aus Agarosegelen. Die Methode kombiniert die Auflösung der die zu isolierende DNA- Bande enthaltende Agarose in einer gesättigten Lösung eines chaotropen Salzes (NaJ) mit einer Bindung der DNA an Glaspartikel. Die an die Glaspartikel fixierte DNA wird anschließend mit einer Waschlösung (20 mM Tris HCI [pH 7,2]; 200mM NaCl; 2 mM EDTA; 50% v/v Ethanol) gewaschen und danach von den Trägerpartikeln abgelöst.

Diese Methode erfuhr bis heute eine Reihe von Modifikationen und wird zum gegenwärtigen Zeitpunkt für unterschiedliche Verfahren der Extraktion und Reinigung von Nukleinsäuren aus unterschiedlichen Herkünften angewendet (Marko, M.A., Chipperfield, R. und Birnboim, H.G., 1982, Anal. Biochem., 121, 382-387).

Darüber hinaus existieren heute weltweit auch eine Vielzahl von Reagenziensystemen, vor allem zur Reinigung von DNA-Fragmenten aus Agarosegelen und für die Isolierung von Plasmid DNA aus bakteriellen Lysaten, aber auch für die Isolierung von längerkettigen Nukleinsäuren (genomische DNA, zelluläre Gesamt-RNA) aus Blut, Geweben oder auch Zellkulturen .

Alle diese kommerziell verfügbaren Kits basieren auf dem hinlänglich bekannten Prinzip der Bindung von Nukleinsäuren an mineralische Träger unter Anwesenheit von Lösungen unterschiedlicher chaotroper Salze und verwenden als Trägermaterialien Suspensionen feingemahlener Glaspulver (z.B. Glasmilk, BIO 101, La Jolla, CA), Diatomenerden (Fa.Sigma) oder auch Silicagele. (Diagen, DE 41 39 664 A1).

Ein für eine Vielzahl unterschiedlicher Anwendungen praktikables Verfahren zur Isolierung von Nukleinsäuren ist in US 5,234,809 (Boom) dargestellt. Dort ist ein Verfahren zur Isolierung von Nukleinsäuren aus nukleinsäurehaltigen Ausgangsmaterialien durch die Inkubation des Ausgangsmaterials mit einem chaotropen Puffer und einer DNA-bindenden festen Phase beschrieben. Die chaotropen Puffer realisieren sowohl die Lyse des Ausgangsmaterials als auch die Bindung der Nukleinsäuren an die feste Phase. Das Verfahren ist gut geeignet, um Nukleinsäuren aus kleinen Probenmengen zu isolieren und findet speziell im Bereich der Isolierung viraler Nukleinsäuren seine praktische Anwendung.

Spezifische Modifikationen dieser Verfahren betreffen den Einsatz von neuartigen Trägermaterialien, welche für bestimmte Fragestellungen applikative Vorteile zeigen (Invitek GmbH WO-A 95/34569).

Entscheidende Nachteile von Verfahren der Isolierung von Nukleinsäuren aus komplexen Ausgangsmaterialien auf der Basis der Inkubation des Ausgangsmaterials mit einem chaotropen Puffer und einer festen Phase bestehen aber u.a. darin, daß der durch die chaotropen Puffer zu realisierende Zellaufschluß nicht für alle Materialien einsetzbar ist bzw. auch für größere Mengen an Ausgangsmaterialien nur extrem ineffizient und unter einem großen Zeitaufwand funktioniert. Darüber hinaus sind mechanische Homogenisierungsverfahren notwendig, wenn z.B. DNA aus Gewebeproben isoliert werden soll. Weiterhin müssen für verschiedene Fragestellungen auch immer verschieden hohe Konzentrationen unterschiedlicher chaotroper Puffer eingesetzt werden. Das Verfahren ist damit in keiner Weise universell einsetzbar.

Probleme, die durch eine ggf. schwierige Lyse des Ausgangsmaterials entstehen, können durch eine Reihe von kommerziell verfügbaren Produkten für die Nukleinsäureisolierung (speziell für die Isolierung genomischer DNA aus komplexen Ausgangsmaterialien) zwar gelöst werden, haben aber den großen Nachteil, daß es sich nicht mehr um ein klassisches "Single Tube"-Verfahren handelt, das das Verfahren gemäß US-Patent kennzeichnet, da die Lyse des Ausgangsmaterials in einem gebräuchlichen Puffer unter Einbeziehung eines proteolytischen Enzyms erfolgt. Die für die nachfolgende Bindung der Nukleinsäuren an z.B. Zentrifugationsmembranen notwendigen chaotropen Ionen müssen nach erfolgter Lyse dem Lyseansatz extra zugegeben werden. Sie können aber nicht Bestandteil des Lysepuffers sein, da die proteinzerstörende Funktion chaotroper Salze bekannt ist und natürlich sofort das für eine effiziente Lyse notwendige proteolytische Enzym ebenfalls zerstören würde.

Trotz einer Reihe von Nachteilen haben sich deshalb die Methoden der Nukleinsäureisolierung unter der Verwendung chaotroper Salze weltweit durchgesetzt und werden mittels kommerziell verfügbarer Produkte millionenfach eingesetzt. Diese Systeme sind extrem einfach in ihrer Durchführung und verfahren immer nach dem Prinzip der Lyse des Ausgangsmaterials, der nachfolgenden Bindung der Nukleinsäure an die feste Phase einer Glas-oder Silikamembran, welche sich in einem Zentrifugationssäulchen an einer Trägersuspension befindet, dem Waschen der gebundenen Nukleinsäuren und der nachfolgenden Elution der Nukleinsäuren mit einem Puffer geringer Ionenstärke.

Alle diese Systeme beruhen auf der Bindung der Nukleinsäuren an die jeweiligen Trägeroberflächen in Anwesenheit chaotroper Salze, d.h. mindestens eine Pufferlösung enthält als Hauptkomponente ein chaotropes Salz. Dies betrifft u.U. schon den Lysepuffer oder bei Systemen unter Einbeziehung proteolytischer Enzyme einen notwendigen Bindungspuffer, welcher nach der erfolgten Lyse des Ausgangsmaterials zugegeben wird.

Die Basis für chaotrope Salze sind die Reihen von Hofmeister zur Aussalzung von negativ geladenen, neutralen oder baischen Eiwweißlösungen. Die chaotropen Salze sind dadurch charakterisiert, Proteine zu denaturieren, die Löslichkeit unpolarer Substanzen in Wasser zu erhöhen sowie hydrophobe Wechselwirkungen zu zerstören. Gerade diese Eigenschaften bewirken nach dem Stand der Technik auch mit Puffersystemen chaotroper Salze die übergeordnete Struktur des wässrigen Millieus zu zerstören um so die Bindung der Nukleinsäuren an ausgewählte feste Phasen zu vermitteln. Die bekanntesten Vertreter zur Nukleinsäureisolierung sind, Natriumperchlorat, Natriumjodid, Kaliumjodid, Guanidiniothiocyanat und Guanidinhydrochiorid. Sie sind jedoch zum einen kostenintensiv und zum anderen teilweise toxisch oder ätzend.

Auf diesem Stand der Technik existieren bis zum heutigen Tag eine sehr große Anzahl an Patentanmeldungen sowie an erteilten Patente, wobei es sich dabei immer um Verfahrensvarianten handelt, wie. z.B. die Verwendung neuer Trägermaterialien oder effizientere Waschpuffer etc., wobei das Grundprinzip immer die Verwendung chaotroper Salze zur Bindung an eine feste Phase aus Silicamaterialien darstellt.

Das physiko-chemische Prinzip der Bindung von Nukleinsäuren an mineralische Träger in Anwesenheit chaotroper Salze gilt in der internationalen Fachwelt als erklärt. Die Bindung der Nukleinsäuren an die Oberflächen der mineralischen Träger bestehe in der Störung übergeordneter Strukturen des wässerigen Millieus, durch welche die Nukleinsäuren an die Oberläche von mineralischen Materialien, insbesondere von Glas- bzw. Silicapartikeln adsorbieren. Zur Störung der übergeordneten Strukturen des wässerigen Millieus ist immer die Anwesenheit chaotroper Ionen erforderlich. Bei hohen Konzentrationen der chaotropen Salze verläuft die Reaktion fast quantitativ. Aufgrund dieser beschriebenenen physikochemischen Erkenntnisse geht deshalb die Fachwelt davon aus, daß alle kommerziell verfügbaren Systeme zur Isolierung von Nukleinsäuren, Pufferkompositionen mit hohen Ionenstärken chaotroper Salze, für die Bindung von Nukleinsäuren an eine Nukleinsäurenbindende feste Phase enthalten müssen.

Um so überraschender war die erfindungsgemäße Erkenntnis, daß Formulierungen, die antichaotrope Salze in einem Lyse/Bindungspuffersystem enthalten, ebenfalls und besser zur Isolierung von Nukleinsäuren aus beliebigen, insbesondere komplexen Ausgangsmaterialien geeignet sind.

Die Erfindung wird gemäß den Ansprüchen realisiert. Gegenstand der Erfindung sind deshalb Formulierungen und Verfahren ohne chaotrope Bestandteile zur Isolierung von Nukleinsäuren unter Bindung an eine feste Phase, insbesondere von DNA aus beliebigen komplexen Ausgangsmaterialien, die ein Lyse/Bindungspuffersystem, das mindestens eine antichaotrope Salzkomponente aufweist, eine feste Phase und an sich bekannte Wasch- und Elutionspuffer enthalten.
Antichaotrope Komponenten im Sinne der Erfindung sind Ammonium-, Cäsium-, Natrium- und/oder Kaliumsalze, vorzugsweise Ammoniumchlorid.

Das Lyse/Bindungspuffersystem weist außerdem an sich bekannte Detergenzien und ggf. Zusatzstoffe auf, so z.B. Tris-HCI, EDTA, Polyvinylpyrrolidone, CTAB, TritonX-100, N-Lauryl-Sarkosin, Natriumcitrat, DTT, SDS und/oder Tween. In einer bevorzugten Ausführungsvariante enthält das Lyse/Bindungspuffersystem zur Bindung an die feste Phase einen Alkohol, wie z.B. Ethanol und Isopropanol und ggf. Enzyme, vorzugsweise Protein-abbauende Enzyme, z.B. eine Proteinase.

Mit der Erfindung kann das dem Stand der Technik entsprechende Prinzip ausgenutzt werden, ein spezifisches Problem der Nukleinsäureisolierung zu lösen, bzw. eine bestehende Variante hinsichtlich bestimmter relevanter Parameter zu optimieren und effektivieren. So ist es für die Durchführung als vollautomatisiertes high-throughput-Verfahren geeignet.

Völlig unerwartet und überraschend können im Unterschied zum bekannten Stand der Technik gemäß vorliegender Erfindung mit Lyse/Bindungspuffersystemen ohne den Bestandteil von chaotropen Salzen Nukleinsäuren, insbesondere genomische DNA an ein mineralisches Trägermaterial gebunden werden und unter den üblichen Reaktionsbedingungen auch eluiert werden.

Es wurde weiterhin gefunden, daß eine Vielzahl ganz unterschiedlicher Salze als Bestandteile von ggf. auch an sich üblichen Lyse/Bindungspuffersystemen für die Bindung von Nukleinsäuren an klassische Trägermaterialien auf Basis von Glas oder Silica ausreichend sind.

Besonders überraschend können die besten Ergebnisse mit Salzen erreicht werden, welche nach ihren chemisch-physikalischen Charakteristika die absolut entgegengesetzten Wirkungen in bezug auf die für die Nukleinsäurebindung bisher verwendeten chaotropen Salze aufweisen. Man kann diese Salze somit als antichaotropisch bezeichnen.

So konnten mit Lyse/Bindungspuffern, deren Hauptkomponente z.B. Ammonimsalze anstelle chaotroper Salze waren (kommerzielle Extraktionskits), bei Extraktionen genomischer DNA aus unterschiedlichen komplexen Ausgangsmaterialien (z.B. Blut, Gewebe, Pflanzen) unter Konstanz der bisher üblichen anderen Reaktionskomponenten, Trägermaterialien sowie bei völlig gleichem Reaktionsablauf mindestens dieselben quantitativen sowie qualitativen Resultate erreicht werden.

Insbesondere das Ammoniumion stellt dabei chemisch-physikalisch in der Hofmeister-Serie das Ion dar, welches absolut entgegengesetzte Merkmale zu den bekannten chaotropen Ionen dieser Serie aufweist.

Allein durch den Austausch der bisher verwendeten chaotropen Salzkomponente durch eine antichaotrope Salzkomponente im Lyse/Bindungspuffer bei bestehender Konstanz aller anderen Parameter, an die an sich bekannten festen Trägeroberflächen ist eine mindestens adäquate quantitative Isolierung von Nukleinsäuren möglich.

Das bedeutet, daß mit einem Salz welches Proteine nicht denaturiert, sondern stabilisiert, welches die Löslichkeit unpolarer Substanzen in Wasser nicht erhöht, sondern absenkt sowie welches hydrophobe Wechselwirkungen nicht zerstört, sondern verstärkt, es genauso möglich ist, Nukleinsäuren, auch aus komplexen Ausgangsmaterialien zu isolieren, aufzureinigen und den an sich üblichen Applikationen zuzuführen.

Mit der vorliegenden Erfindung wird ein neuartiger, alternativen Mechanismus zur Bindung von Nukleinsäuren an feste vorzugsweise mineralische Trägermaterialien und auf dieser Basis ein universell anwendbares neuartiges Verfahren der Isolierung von Nukleinsäuren aus komplexen Ausgangsmaterialien bereitgestellt.

Die Erfindung ermöglicht somit über die Verwendung der neuartigen Kompositionen von Lyse/Bindungspuffern auf der Basis von antichaotropen Salzen für die Nukleinsäurenisolierung, speziell für die Isolierung genomischer DNA, basierend auf der Bindung der Nukleinsäuren an die an sich gebräuchlichen unterschiedlichen festen Phasen aus Silica bzw. Glasmaterialien, die Nutzung eines alternativen Chemismus als essentiellem Bestandteil von entsprechenden Testkits (Formulierungen).

Das erfindungsgemäße Verfahren unter Einbeziehung von antichaotropen Salzen folgt dabei den von der praktischen Laborroutine bekannten Verfahrensabläufen für die Nukleinsäureisolierung und ist charakterisiert durch:
1. Lyse des Ausgangsmaterials
2. Bindung der Nukleinsäuren an eine feste Phase (Zentrifugationssäule oder Suspension)
3.Waschen der gebundenen Nukleinsäuren
4. Elution der Nukleinsäuren mit einem an sich bekannten Niedrigsalzpuffer.

Die Erfindung ermöglicht eine hocheffiziente und schnelle Isolierung von Nukleinsäuren, besonders genomische DNA aus jedem beliebigen und ggf. komplexen Ausgangsmaterial. Die für die Bindung notwendigen antichaotropen Ionen können selbst bei Einbeziehung von proteolytischen Enzymen Bestandteil des Lyse/Bindungspuffers sein. Das erfindungsgemäße Verfahren ist damit einfach handhabbar und universell einsetzbar.

Die Realisierung der Nukleinsäureisolierung, insbesondere von DNA, aus beliebigen Ausgangsmaterialien erfolgt durch die Inkubation des die Nukleinsäure enthaltenden Ausgangsmaterials ohne Verwendung chaotroper Substanzen, die mit dem
- Lyse/Bindungspuffersystem, welches eine wässerige Lösung umfaßt, die eine antichaotrope Salzkomponente, mindestens ein Detergenz, ggf. Zusatzstoffe und ggf. ein Enzym aufweist,
- und einer beliebigen festen Phase, vorzugsweise Glafaservliese, Glasmembranen, Gläser, Zeolithe, Keramik sowie andere Siliciumträger in Kontakt gebracht werden,
wodurch die Lyse des Ausgangsmaterials und die nachfolgende Bindung der DNA an die feste Phase erfolgt. Anschließend wird die gebundene Nukleinsäure nach an sich bekannten Methoden gewaschen und die DNA von der festen Phase gelöst.

Bei bestimmten Extraktionsprotokollen kann der Lyseansatz ggf. mit einem zusätzlichen Detergenz, einem Alkohol oder einem Detergenz/Allkohol-Gemisch versetzt werden.

Bevorzugte Ausgangsmaterialien sind kompakte Pflanzenmaterialien, wie z.B. Früchte; Samen; Blätter; Nadeln etc., klinisch relevanten Proben, wie z.B. Vollblut; Gewebe, Mikrobioptate, paraffinierte Materialien, ercp-Proben, Tupfermaterial von Abstrichen, Lebensmittel, wie z.B. Fisch, Wurst, Konserven, Milch, forensischen Proben, wie z.B. Haarwurzeln, Zigarettenkippen, Blutspuren und andere Proben, die DNA enthalten.

Bevorzugte Ionen im Sinne der Erfindung sind die in der Hofmeister-Serie dargestellten antichaotropen Ammoniumionen, Cäsiumionen sowie Kalium- und Natriumionen oder Kombinationen dieser Ionen, vorzugsweise Ammoniumchlorid. Zur Lyse/Bindung werden sie in lonenstärken von 0,1 M bis 8 M eingesetzt.

Für die Bindung der Nukleinsäuren, besonders von DNA an die festen Träger reichen dabei schon geringe Konzentrationen an diesen Salzen von vorzugsweise ≤ 1 M, bei bestimmten Applikationen bevorzugt sogar Konzentrationen ≤ 0,5 M, wobei für die quantitative Isolierung von Nukleinsäuren aus größeren Mengen an Ausgangsmaterialien höhere lonenkonzentrationen erfolgreich sind.

Es können durch die Verwendung der antichaotropen Salze, die proteinstabilisierend wirken, als essentielle Bestandteile eines Lysepuffers in einer bevorzugten Ausführungsform der Erfindung auch proteolytische Enzyme, wie z.B Proteinase K, zur Unterstützung und Effektivierung des Lyseprozesses, wodurch für den notwendigen Zellaufschluß auch hohe Ionenstärken der antichaotropen Salze z.B. 5 M zugesetzt werden, so daß eine quantitative Isolierung von Nukleinsäuren ermöglicht wird.

Puffersysteme des Standes der Technik mit den bekannten chaotropen Salze können bei den notwendigen hohen Ionenstärken, wie sie allgemein für eine quantitative Isolierung von Nukleinsäuren gefordert werden, keine proteolytischen Enzyme enthalten. Sie müssen somit immer nachträglich für die Bindung der Nukleinsäuren an die feste Phasen zugesetzt werden.

Als Detergentien in den erfindungsgemäßen Lysepuffern/Bindungspuffern werden bevorzugt anionische, kationische oder neutrale, wie z.B. SDS, Triton X-100, Tween oder CTAB eingesetzt.

Nach der erfolgten Lyse des Ausgangsmaterials wird die Suspension ggf. durch einen kurzen Zentrifugationsschritt von noch nicht vollständig lysierten Bestandteilen abgetrennt und mit dem DNA-bindenden Material direkt inkubiert bzw. wie schon beschrieben nach Zugabe mit einem zusätzlichen Detergenz, einem Alkohol oder einem Detergenz/Alkohol-Gemisch mit der festen Phase inkubiert. Gegebenenfalls befinden sich im Lysepuffersystem zusätzlich geringe Konzentrationen (< 50 mM ) an EDTA und/oder Tris-HCl. Für die Isolierung von DNA aus sehr stark verunreinigten Ausgangsmaterialien erfolgt bevorzugt auch der Zusatz von 2-4% Polyvinylpyrrolidon oder anderen bekannten Substanzen zum Puffersystem zur selektiven Bindung von inhibitorischen Komponenten.

Als Bindungsmaterialien für die zu isolierende DNA haben sich z.B. kommerziell verfügbare Glasfaservliese in Zentrifugationssäulen, Siliziumverbindungen wie SiO₂ unterschiedlicher Teilchengröße hervorragend bewährt. Damit können alle die Materialien verwendet werden, welche für die Isolierung von Nukleinsäuren mittels chaotroper Puffer genutzt werden.

Nach der Inkubation mit dem DNA-bindenden Material erfolgt die Abtrennung des Lysates vom Bindungsmaterial durch einen kurzen Zentrifugationsschritt. Nachfolgend wird in an sich bekannter Weise mit einem Waschpuffer z.B. bestehend aus mindestens 50% Ethanol und gegebenenfalls einer geringen Salzkonzentration z.B. NaCl gewaschen, das Trägermaterial wird getrocknet und die gebundene DNA mittels eines an sich bekannten Niedrigsalzpuffers (Tris-HCl; TE; Wasser) und bei einer bevorzugten Temperatur von 50-70°C eluiert.

Eine weitere Ausführungsvariante der Erfindung besteht darin, daß zur Lyse von schwer aufschließbaren Ausgangsmaterialien, z.B. kompakten Gewebeproben, Haarwurzeln bzw. zur Optimierung der Lyseeffizienz und zur Reduzierung notwendiger Lysezeiten der Zusatz von proteolytischen Enzymen, vorzugsweise Proteinasen, wie z.B. Proteinase K, erfolgt.

Die Erfindung ermöglicht somit auf neuartigen Kombinationen antichaotroper Salze als essentiellen Bestandteilen von Lysepuffermixturen universell einsetzbare Verfahren zur Isolierung von Nukleinsäuren, insbesondere DNA, aus allen DNA enthaltenden Ausgangsmaterialien wie auch aus beliebigen Mengen an unterschiedlichsten Ausgangsmaterialien, wobei alle die bisher eingesetzten Trägermaterialien und deren Ausführungen genauso effizient eingesetzt werden können, wie auch die bisher praktizierten Vorschriften der Isolierung von Nukleinsäuren identisch nutzbar sind.

In seiner allgemeinsten Anwendungsvariante kann mittels des erfindungsgemäßen Verfahrens aus allen dem Stand der Technik entsprechenden für eine DNA-Extraktion ausgewählten komplexen Ausgangsmaterialien eine Nukleinsäureextraktion durchgeführt werden, d.h. mittels des neuen universellen Puffersystem kann die hocheffiziente Lyse und nachfolgende Nukleinsäurebindung an einen mineralischen Träger aus kompakten Pflanzenmaterialien (z.B. Früchte; Samen; Blätter; Nadeln etc.), aus klinisch relevanten Proben (z.B. Vollblut; Gewebe, Mikrobioptate, paraffinierte Materialien, ercp-proben, Tupfermaterial von Abstrichen), aus Lebensmitteln (z.B. Fisch, Wurst, Konserven, Milch), aus forensischen Proben (z.B. Haarwurzeln, Zigarettenkippen, Blutspuren) wie auch aus anderen Ausgangsmaterialien erfolgreich, extrem einfach und sehr schnell durchgeführt werden.

Ein weiterer Vorteil des Verfahrens besteht auch darin, daß die Isolierung von DNA dabei sowohl aus extrem geringen Ausgangsmaterialien (z.B. Isolierung von DNA aus 1 µl Vollblut; Haarwurzel, Mikrobiopsie < 1 mg) als auch aus sehr großen Mengen an Ausgangsmaterialien wie z.B. 50 ml Vollblut; 1 g Gewebematerial, <1g Pflanzenmaterial hocheffizient durchgeführt werden kann.

Weitere Vorteile der Ablösung chaotroper Salze durch antichaotrope Salze bestehen darin, daß die eingesetzten Puffer aufgrund des Fehlens der chaotropen Chemikalien auch nicht mehr toxisch oder ätzend wirken.

Neben einer allgemeinsten Ausführungsvariante erlauben Optimierungen des Extraktionsverfahrens bezogen auf spezifische Applikationen sogar eine fast quantitative Isolierung der in der Ausgangsprobe enthaltenenen DNA-Mengen. Erstaunlicherweise können mit dem erfindungsgemäßen Verfahren ohne die nach dem Stand der Technik eingesetzten chaotropen Ionen hoher Konzentration für eine DNA-Bindung, höhere DNA-Ausbeuten erzielt werden, als dies mit kommerziell verfügbaren und hochoptimierten Extraktionskits bisher möglich ist.

Eine Auswahl diesbezüglicher Vergleichsergebnisse mit kommerziell verfügbaren Extraktionskits sind in den Ausführungsbeispielen dargestellt. Diese Ergebnisse demonstrieren eindeutig die Potentiale der Erfindung.

Neben der Isolierung von DNA aus allen DNA enthaltenden komplexen Ausgangsmaterialien, ermöglicht eine weitere Ausführungsvariante des erfindungsgemäßen Verfahrens auch die Isolierung von Plasmid-DNA aus bakteriellen Lysaten hocheffizient und ohne den Einsatz von nach dem Stand der Technik zur Bindung der Plasmid DNA an mineralische Trägermaterialien an sich notwendigen chaotropen Salzen. So wird nach den dem Fachmann bekannten Verfahrensschritten der Isolierung von Plasmid-DNA mittels alkalischer Lyse die notwendige sog. Neutralisationsreaktion mittels der klassischen Solution III (Maniatis und Sambroek) durchgeführt und diese Solution III realisiert in einer damit bestehenden Dualfunktion auch gleichzeitig die Bindung der Plasmid-DNA an die an sich gebräuchlichen festen Träger. Für die Bindung der Plasmid-DNA bedarf es somit nicht dem gebräuchlichen Zusatz eines chaotropen Guanidiniumhydrochlorides.

Die gebundene Plasmid-DNA wird auch in an sich bekannter Art gewaschen und vom Trägermaterial eluiert. Das Verfahren eignet sich für die Isolierung von Plasmid-DNA aus allen Anwendung findenden Ausgangsmengen (Mini bis Giga). Die erhaltenen Ausbeuten an Plasmid-DNA sind dabei gegenüber mit herkömmlichen kommerziell verfügbaren Verfahren isolierten Ausbeuten identisch. Das erfindungsgemäße Verfahren ist jedoch in seiner Herstellung sehr viel preiswerter als alle anderen bekannten Systeme, denn chaotrope Salze sind sehr kostenintensiv.

Das Verfahren unter Verwendung antichaotroper Salze eignet sich somit auch hervorragend für die Konzipierung von automatisierbaren Systemen für die Plasmidisolierung, bei welchen bekanntermaßen der Preis/Präparationen ein entscheidendes Auswahlkriterium ist.

Die erfindungsgemäßen Formulierungen ermöglichen in überraschender Weise den Zugang zu weiteren hochinteressanten und neuartigen Applikationen auf dem Gebiet der Nukleinsäureisolierung und Diagnostik.

In einer weiteren Ausführungsform der Erfindung sind die vorliegenden neuen Lyse/Bindungspuffersysteme, die mindestens eine antichaotrope Salzkomponente aufweisen, in der Lage Nukleinsäuren an feste Phasen zu binden, die eine negativ geladene Oberfläche besitzen oder Oberflächen, die ein negatives Ladungspotential aufweisen.

Aus dem Stand der Technik sind Verfahren und Mittel zu Nukleinsäurereinigung bekannt, wobei die Nukleinsäurebindung an chemisch modifizierte feste Phasen erfolgt (United States Patent: 5,523,392; Purification of DNA on Aluminium Silicates and Phosphosilicates; United States Patent: 5,503,816; Silicates Compounds for DNA Purification; United States Patent:5,674,997; DNA purification on modified Siligates; United States Patent:5,438,127; DNA Purification by solid phase extraction using a PCl₃ modified glass fiber membrane; United States Patent:5,606,046: DNA purification by solid phase extraction using trifluormetric acid washed glass fiber membrane; United States Patent: DNA purification by solid phase extraction using glass fiber membrane previously treated with trifluoroacetic acid, and then with fluoride ion, hydroxyd ion, or BCL_{3:} United States Patent:5,610,291: Glass fiber membranes modified by treatment with SiCl₃, AlCl₃, or BCl₃ and washing with NaOH to set as a DNA adsorbant; United States Patent: 5,616,701: DNA purification by solid phase extraction using a hydroxide-washed glass fiber membrane; United States Patent:5,650,506: Modified glass fiber membranes useful for DNA purification by solid phase extraction).

Bedingung für diese Nukleinsäurebindung ist dabei immer, daß die für die Bindung verwendeten Membranen durch chemische Modifizierungsreaktionen mit positiven Ionenladungen dotiert werden. Damit liegt auf der Hand, das es zwischen der positiv geladenenen Oberfläche der eingesetzten Membranen und der negativen Ionenladung des Phosphatrückgrats von Nukleinsäuren durch Coulombsche Wechselwirkungen eine Bindung ergeben wird. Insofern wird das der Fachwelt hinlänglich bekannte Prinzip der Bindung von Nukleinsäuren an positive geladene feste Phasen genutzt, welches bekanntermaßen z.B. für DNA/RNA-Blotting-Techniken an positiv geladenenen Nylon-Filtern schon viele Jahre eine Standardapplikation darstellt.

Ein ganz wesentlicher Nachteil dieser beschriebenen Verfahren besteht allerdings darin, daß diese nicht zur Nukleinsäureisolierung geeignet sind, d.h. es ist komplett unmöglich Nukleinsäuren aus komplexen Ausggangsmaterialien zu isolieren. Ausgangsmaterial sind immer schon bereits isolierte Nukleinsäuren, welche wie in den zitierten US-Patentschriften dargestellt, in an sich bekannter Weise isoliert werden müssen. Insbesondere ein Aspekt erscheint dem Fachmann dabei unklar. Die beschriebenen Bindungsbedingungen (Bindung unter physiologischen Pufferbedingungen) und Elutionsbedingungen sind identisch. Es ist nicht zu ersehen wie unter denselben Pufferbedingungen zur Bindung der Nukleinsäuren an die positiv geladene Membran auch wieder die Nukleinsäuren von der Membran abgelöst werden können.
Letzlich können die dargestellten Mittel und das dazugehörende Verfahren eine nur sehr enge praktische Anwendung besitzen. Bekannt sind auch synthetisch hergestellte Oligonukleotide an die positiven Oberflächen zu binden. Dies erfolgt dabei wiederum unter Ausnutzung Coulombscher Wechselwirkungen, d.h. auf der Basis der Verknüpfung postiver und negativen Ladungen z.B. über modifizierte Oligonukleotide (Verknüpfung mit Aminolinkern oder Phospatlinkern). Auch diese Varianten ermöglichen nicht die Isolierung von Nukleinsäuren aus komplexen Ausgangsmaterialien.

Wie ausführlich dargestellt, existieren alternative Formen der Nukleinsäurebindung zur Reinigung an Membranen mit ausreichender positiver Ladung, die keine Verfahren zur Isolierung von Nukleinsäuren darstellen. Die Bindung der Nukleinsäuren erfolgt durch Coulombscher Kräfte, basierend auf Wechselwirkungen zwischen positiven Ionenladungen der Membranen und der negativen Ionenladungen des Nukleinsäurenrückrats. Dieses Prinzip erscheint damit logisch erklärbar.

Basierend auf der erfindungsgemäßen Isolierung von Nukleinsäuren aus komplexen Ausgangsmaterialien mit antichaotropen Salze wurde ein überraschendes Phänomen gefunden. So zeigte sich, daß sich auch negativ geladene Oberfläche oder Oberflächen, die in ein negatives Ladungspotential überführt werden können, für die Bindung von Nukleinsäuren unter Verwendung der erfindungsgemäßen Lyse/Bindungspuffersysteme eignen. Allgemein konnte eine solche Möglichkeit nicht erwartet werden, da keine Bindung sondern eine Abstoßung auf Grund gleicher Ladungspotentiale eintreten müsste.

Die erfindungsgemäß eingesetzten negativ funktionalisierten Oberflächen oder mit potientielle negativen Modifizierungen ausgestattete Oberflächen werden nach an sich bekannten Verfahren erzeugt. Als geeignet hat sich z.B. die photochemische Kopplung einer Acetylgruppe, Carboxylgruppe oder Hydroxylgruppe an die Oberfläche eines Reaktionsgefäßes gezeigt.

Mit der vorliegenden Verfahrensvariante ermöglichen sich völlig neue Perspektiven für eine komplexe Nukleinsäureanalytik. Es zeigte sich nämlich, daß für die Bindung der Nukleinsäure an negative oder potentiell negative Oberflächen die Nukleinsäure wie bei allen bisher beschriebenen Varianten nicht schon isoliert sein muß. Die Bindung erfolgt aus dem Lysereaktionsansatz heraus, d.h. die die Nukleinsäure enthaltende Ausgangsprobe wird lysiert und die frei werdenen Nukleinsäuren binden an die negativ geladene Oberfläche (z.B. an eine Mikrotestplattenkavität oder ein Eppendorf-Reaktionsgefäß).

Durch die erfindungsgemäße Verfahrensvariante können nunmehr völlig neuartige "Single Tube" und Einschritt-Verfahren zur Isolierung von Nukleinsäuren aus komplexen Ausgangsmaterialien realisiert werden. Solche Verfahren bieten in ihrem Anwendungsspektrum für die Nutzer riesige Vorteile (Einfachheit; Billigkeit; Reduzierung von Abfall, Schnelligkeit, Routinetauglichkeit, Automatisierbarkeit u.a.m.)

Eine weitere Anwendung dieser Verfahrensvariante besteht außerdem darin, nicht nur die Extraktion der Nukleinsäuren in einer Reaktionskavität zu realisieren, sondern auch eine folgende Targetamplifikation und ggf. nachfolgende Analytik im selben Reaktionsgefäß durchzuführen, ggf. Hybridisierungsreaktionen durchzuführen oder Sequenzierungen an festen Phasen ablaufen zu lassen.

Auf dieser Basis wird z.B. ein 0.5 ml Eppendorf PCR-Reaktionsgefäß mittels der Fachwelt bekannter Techniken mit einer negativ geladenen oder potentiell negativen funktionalen Gruppe modifiziert. Dazu eiget sich z.B. die photochemische Kopplung einer Acetylgruppe, Carboxylgruppe oder Hydroxylgruppe an die Oberfläche des Reaktionsgefäßes. In das Reaktionsgefäß wird dann die für die Nukleinsäurenisolierung ausgwählte Probe gegeben (z.B. Vollblut) und mit einem Lysepuffer, enthaltend die antichaotrope Salzfraktion z.B. Ammoniumchlorid, ein Detergenz und ein proteolytisches Enzym versetzt und das Gefäß bei 70°C für 5 min inkubiert.

Zur Maximierung der Nukleinsäurenbindung kann nach der Lyse des Ausgangsmaterials noch ein Detergenz/Alkohol-Gemisch pipettiert werden. Der Ansatz wird dann für kurz inkubiert und nachfolgend aus dem Reaktionsgefäß abgegossen. Die Nukleinsäure befindet sich nun an der funktionalisierten Oberfläche des Reaktionsgefäßes gebunden und wird nachfolgend mit einem alkoholischen Waschpuffer kurz gespült und der Alkohol wird durch Inkubation bei z.B. 70°C entfernt. Die Elution der gebundenen Nukleinsäuren erfolgt weiter fachgemäß durch die Zugabe eines Niedrigsalzpuffers (z.B. 10 mM Tris-HCl) in das Reaktionsgefäß und eine kurze Inkubation (z.B. 2 min ) bei z.B. 70°C. Die Nukleinsäure ist so für nachfolgende Verwendungen verfügbar.

Wie dargestellt laufen alle Reaktionen der Nukleinsäureisolierung aus einem komplexen Ausgangsmaterial in einem Reaktionsgefäß ab; d.h. Lyse des Ausgangsmaterials, Bindung der Nukleinsäuren; Waschen der gebundenen Nukleinsäuren und Elution der Nukleinsäuren werden in und mit einem Reaktionsgefäß realisiert.

Die den momentan weltweit am Häufigsten genutzten Extraktionskits der Fa. Qiagen benötigen für die Abfolge von Lyse, Bindung, Waschen und Elution jeweils eine Filterkartusche und mindestens 4 separate Reaktionsgefäße eingeschlossen sind desweiteren multiple Zentrifugationsschritte.

Das erfindungsgemäße Verfahrensvariante erlaubt im Gegensatz dazu die Extraktion der Nukleinsäure ohne einen einzigen Zentrifugationsschritt. Daraus läßt sich auch ein enormer zeitlicher Vorteil ableiten. Diese Vorteile beziehen sich auch auf die beschriebenen Nukleinsäureextraktionsverfahren des zitierten US 5,234,809 von Boom.

Neben der möglichen Extraktion von Nukleinsäure kann die gebundene Nukleinsäure aber auch an der Oberfläche des beschriebenen 0.5 ml Reaktionsgefäßes verbleiben und z.B. nachfolgend durch Zugabe eines kompletten PCR-Raktionsmixes (Primer, Nukleotide, Polymerasepuffer, Taq Polymerase, Magnesium) gleich für eine PCR-Applikation verwendet werden, d.h Extraktion und Amplifikation laufen dann im selben Reaktionsgefäß ab.

Diese Beispiele illustrieren die enormen Vorteile und breite Anwendbarkeit, die aus der Erfindung ableitbar sind. Sie ermöglicht in einer Ausführungsvariante den kompletten Vorgang von Probenvorbereitung über Amplifikation und ggf. auch Analytik in z.B.einer Reaktionskavität. Damit ergeben sich mit der Bereitstellung von modifizierten Reaktionsgefäßen (oder auch anderen festen Oberflächen) und den geeigneten Lyse/Bindungspuffern neue Standards in molekularbiologisch und vor allem Nukleinsäure-Diagnostik bearbeitenden Laboratorien, wobei durch die neuen potentiellen applikativen Lösungen vor allem auch die hinlänglich bekannten Probleme der Probenkontaminationen drastisch reduziert werden.

Ein weiterer Vorteil und auch eine weitere Applikation besteht darin, daß die oberflächenfixierten Nukleinsäuren an der Oberfläche auch zumindest längere Zeit stabil fixiert und somit für eine spätere Bearbeitung verfügbar sind, d.h die PCR-Reaktion muß sich nicht zwingend sofort der Extraktion anschließen. Ein weiteres Anwendungsfeld ist die vollautomatisierte Nukleinsäureextraktion und ggf. Analytik unter Verwendung der hier beschriebenen mit negativen oder mit potentiell negative Ladungen tragenden Oberflächen, vorzugsweise Plastikoberflächen geeigneter Reaktionskavitäten z.B. Mikrotestplatten).

Die erfindungsgemäßen Lyse/Bindungspuffersysteme mit den antichaotropen Salzen als Hauptkomponenten einschließlich ggf. eines proteolytischen Enzyms können auch als feste Formulierung bereitgestellt. Dazu werden die Mischungen aus Salzen und Detergenzien, Zusatzstoffen und ggf. Enzymen in gebräuchlichen Rektionsgefäßen aliquotiert und für mehrere Stunden bei 95 °C inkubiert oder nach an sich bekannten Verfahren lyophilisiert und so in eine feste Formulierung überführt.

Diese feste Formulierungen in fertigen komplexen Reaktionsmixen für die Nukleinsäureisolierung sind langzeitlagerstabil, d.h auch die biologische Aktivität der proteolytischen Enzymkomponente bleibt bei einer Langzeitlagerung (siehe Ausführungsbeispiel) bestehen. Die Herstellung der festen Formulierung von Lysepuffermixen erfolgte dabei ohne den Zusatz von an sich bekannten protektiven Zusatzstoffen, einfach durch eine Kältelyophilisierung.

Alle kommerziell angebotenen Testkits zur Nukleinsäurenextraktion enthalten die notwendigen Komponenten einzeln, bestimmte Lösungen müssen durch den Anwender erst hergestellt werden und darüber hinaus sind die Lösungen in ihrer Haltbarkeit eingeschränkt. Ein weiterer Nachteil besteht darin, daß der Nutzer während der Isolierung von Nukleinsäuren unter Verwendung z.Z. gebräuchlicher Testkits multiple Pipettierschritte für verschiedene Einzellösungen einhalten muß. Dies erhöht vor allem im Bereich der medizinischen Diagnostik das Kontaminationsrisiko dramatisch. Nachteilig ist ferner auch,daß durch die z.B. existierenden Beladungsgrenzen von weit gebräuchlichen Zentrifugationssäulchen, welche hauptsächlich für die Nukleinsäureisolierung angewendet werden, auch die Menge des Ausgangsmaterials stark begrenzt ist. Dies liegt darin begründet, das zum Ausgangsmaterial noch die für die Extraktion notwendigen Lyse- und Bindungspuffer zugegeben werden müssen.

Durch die Bereitstellung einer festen Formulierung als lagerstabiler Lysemix auf der Basis antichaotroper Salze werden alle die bestehenden Probleme in ganz einfacher Form gelöst. Diese Formulierung hat folgende Vorteile:
1. Langzeitlageriung von. "Ready to use" Lysepuffermixen
2. Stabilisierung von proteolytischen Enzymen in fertigen Lysemixturen und deren Langzeitlagerung
3. Einsatz von größeren Mengen an Ausgangsmaterialien bei gleicher Dimensionierung von bestehenden Zentrifugationssäulchen (z.B. Verdreifachung der Ausganngsmenge)
4. Reduzierung von Kontaminationsrisiken durch die Reduzierung von Pipettierschritten und Lösungen
5. Probenaufnahme im fertigen Lysemix auch außerhalb des Labors und deren ggf. Langzeitlagerung
6. stabiler Probenversand und Kühlung

Die fertigen festen, stabilen Lysepuffermixe bestehend aus einer Vielzahl von Einzelkomponenten; einschließlich ggf. proteolytischen Enzymen sind einfach handhabbar (auch von Personen ohne Sachkenntnisse), da die Reaktion einfach durch Zugabe einer Probe, die die zu isolierende Nukleinsäure enthält, gestartet wird. Darüber hinaus kann davon ausgegangen werden, daß die Mixturen entsprechend ihrer Inhaltstoffe Haltbarkeiten von mindestens 6 Monaten aufweisen, wodurch auch ein Transport der Probe bei Umgebungstemperatur kein Problem mehr darstellt.

Der Vorteil der festen Formulierungen basiert darauf, daß für die Lyse von Nukleinsäuren (NAs) enthaltenden Probenmaterialien eine diese NAs enthaltende Probe lediglich in das Reaktionsgefäß mit dem enthaltenden lagerstabilen Lysepuffer überführt wird und ggf. durch die Zugabe von Wasser, die Probe im jeweiligen Reaktionsgefäß lysiert wird. Aufwendige und kontaminationsbelastende multiple Pipettierschritte entfallen gänzlich. Vor allem für die Sammlung und Aufarbeitung von klinischen und forensischen Proben unter Feldbedingungen sind durch die erfindungsgemäße Formulierung die bekannten Probleme gelöst und es steht eine einfach zu handhabende Formulierung zur Verfügung.

Überaschenderweise zeigte sich dann ebenfalls in der praktischen Durchführung, daß nach Zugabe des zu lysierenden Ausgangsmaterials ggf. bei Zugabe einer festen Probe nach Zugabe von H₂O die feste Formulierung unter Standardreaktionsbedingungen problemlos wieder in eine flüssige Phase überführbar ist.

### Zusammenfassend sei festzustellen:

Gegenstand der Erfindung ist die Verwendung antichaotroper Salze in Formulierungen ohne chaotrope Bestandteile zur Isolierung von Nukleinsäuren unter Bindung an eine feste Phase, insbesondere von DNA aus beliebigen komplexen Ausgangsmaterialien. Die Formulierungen enthalten Lyse/Bindungspuffersysteme, die mindestens eine antichaotrope Salzkomponente aufweisen, eine feste Phase und an sich bekannte Wasch- und Elutionspuffer.
Das Lyse/Bindungspuffersystem kann als wässerige Lösung vorliegen oder als feste Formulierung in einsatzfertigen Reaktionsgefäßen.
Als feste Phase können alle Trägermaterialien fungieren, die zur Isolierung mittels chaotroper Reagentien Anwendung finden, vorzugsweise Glafaservliese, Glasmembranen, Siliciumträger und Aerosile oder Trägermaterialien, die eine negativ geladene Oberfläche besitzen oder chemisch modifizierte Oberflächen aufweisen, die in ein negatives Ladungspotential besitzen.

Gegenstand der Erfindung ist ferner ein Verfahren zur Isolierung von Nukleinsäuren, insbesondere von DNA, aus beliebigen komplexen Ausgangsmaterialien unter Verwendung der genannten Formulierungen, das durch Lyse des Ausgangsmaterial, Bindung der Nukleinsäuren an ein Trägermaterial, Waschung der am Träger gebundenen Nukleinsäuren und Elution der Nukleinsäuren gekennzeichnet ist.

Aufgrund der erzielten DNA-Qualität ist es auch zur präparativen Isolierung und Aufreinigung für DNA zum Einsatz in der Gentherapie gut geeignet.

Gegenstand der Erfindung sind auch lagerstabile und einsatzfertige feste Formulierung von Lysepuffersystemen für die Nukleinsäureisolierung auf der Basis antichaotroper Salzen, die als "Ready-to-Use"-Mixe einsatzfertig in konventionellen Reaktionsgefäßen vorliegen. Die festen Formulierungen der Lysepufferansätze werden durch die Zugabe von lediglich der Probe (bei flüssigen Proben wie z.B. Vollblut, Speichel, Zellsuspensionen, Serum, Plasma, Liquor), bei festen Ausgangsmaterialien wie Gewebe, Haarwurzeln, Blutspuren an festen Oberlächen, Zigarettenkippen deparaffiniertebGewebe u.a.m. zusätzlich durch Zugabe von Wasser aktiviert und realisieren die Lyse des Ausgangsmaterials. Nach erfolgter Lyse des Ausgangsmaterials wird der Lyseansatz in an sich bekannter Weise ggf. nach Zugabe einer ethanolische Lösung bzw. eines Alkohol/Detergenz-Gemisches mit den Verwendung findenden nukleinsäurebindenden festen Phasen jeglicher Form (Suspension, Zentrifugationssäulchen) inkubiert. Die nachfolgende Bindung der Nukleinsäuren an die jeweiligen festen Phasen, das Waschen der gebundenen Nukleinsäuren und die finale Elution erfolgen wie schon beschrieben nach dem Stand der Technik.
Mit diesen festen Formulierungen sind neuartige Lösungen vor allem für die Anwendungsgebiete jeglicher Form von Nukleinsäurendiagnostik gegeben.

Hervorgehoben werden soll noch einmal, daß die Erfindungsvariante in einem Einschritt-Verfahren und in einem "Single Tube"-Verfahren die Isolierung von Nukleinsäuren aus komplexen Ausgangsmaterialien, ggf. Targetamplifikationen und ggf. nachfolgende Analytik des amplifizierten Nukleinsäureabschnittes ermöglicht. Ausgangsmaterial muß dabei nicht eine schon isolierte Nukleinsäure sein, sondern ist das komplexe die Nukleinsäure enthaltende Ausgangsmaterial. Die für die Bindung der Nukleinsäure benötigte Oberfläche enthält negative oder potentiell negative funktionale Gruppen. Die Bindung der Nukleinsäure wird in einem Lyse/Bindungspuffer realisiert, wobei die für die Bindung der negativ geladenen Nukleinsäure an die negative funktionalisierte Oberfläche benötigten Ionen aus antichaotropen Salzen stammen.
Damit sind realisierbar:
1. "Single Tube"-Verfahren zur Isolierung von Nukleinsäuren aus komplexen Ausgangsmaterialien
2. "Single Tube"-Verfahren zur Isolierung von Nukleinsäuren aus komplexen Ausgangsmaterialien und nachfolgende Targelvervielfältigung
3. "Single Tube"-Verfahren zur Isolierung von Nukleinsäuren aus komplexen Ausgangsmaterialien, nachfolgende Targetvervielfältigung und nachfolgende Analytik des vervielfältigten Nukleinsäureabschnittes.

Das bedeutet sowohl Nukleinsäure-Isolierung aus unterschiedlichsten DNA enthaltenen Ausgangsmaterialien ggf. Targetvervielfältigung und ggf. Analytik finden in ein und der selben Reaktionskavität oder ggf. auf ein und der selben Reaktionsoberfläche statt.

Die erfindungsgemäßen Formulierungen und das universelle Verfahren zur Bindung von Nukleinsäuren an festen Phasen zur Isolierung, Aufreinigung und nachfolgenden komplexen molekularen Analytik von Nukleinsäuren aus beliebigen Ausgangsmaterialien und Mengen, welche Nukleinsäuren enthalten, stellen eine neuartige Plattformtechnologie für die Entwicklung von integrativen vollautomatisierbaren genanalytischen Systemen dar, die es ermöglichen. Probenvorbereitung, Targetvervielfälltigung und Targetanalytik in einer Reaktionskavität zu realisieren.

Die Erfindung wird anschließend an Ausführungsbeispielen näher erläutert.

### 1. Isolierung genomischer DNA aus unterschiedlichen Pflanzenmaterialien

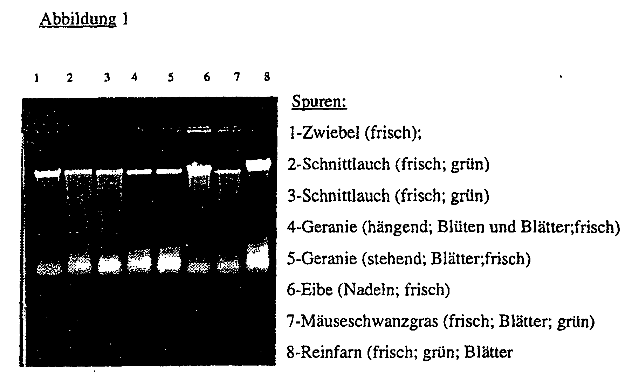

Jeweils 50-100 mg des pflanzlichen Ausgangsmaterials wurden unter flüssigem Stickstoff zermörsert und nachfolgend in ein 1.5 ml Eppendorf-Reaktionsgefäß überführt.
Zugabe von 500µl Lysepuffer (2% CTAB; 2% Polyvinylpyrolidon, 10mM Tris-HCl; 20mM EDTA und 1,3 M Ammoniumchlorid) und Inkubation für mindestens 30 min bei 65°C.
Abzentrifugieren unlysierter Komponenten und Mischen des Überstandes mit 200µl Isopropanol.
Überführen der Lösung auf eine Zentrifugationssäule mit einer Glasfasermembran (Micro Spin Säule; Fa. LIDA).
Zentrifugation für 2 min bei 12.000 rpm. Verwerfen des Filtrates und 2 maliges Waschen der Membran mit einem Waschpuffer (50 mM NaCl; 10 mM Tris HCl; 1 mM EDTA; 70% v/v Ethanol).
Nach Ethanolentfernung durch einen kurzen Zentrifugationsschritt (12.000 rpm für 2min) Zugabe von 200µl eines Elutionspuffers (10 mM Tris-HCI; pH 8,7) und Elution der DNA durch Zentrifugation für 1 min bei 10.000 rpm.
Jeweils 20µl der eluierten DNA wurden auf ein Agarosegel geladen und nach Ethidiumbromidfärbung dargestellt (Abbildung 1)

### 2. Simultane Isolierung genomischer DNA aus unterschiedlichen Ausgangsmaterialien mit einem Universalpuffersystem

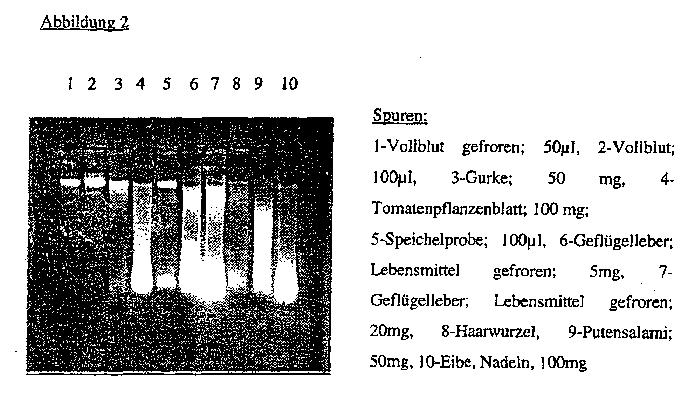

Für die Isolierung wurden folgende Proben eingesetzt:
1-Vollblut gefroren; 50µl, 2-Vollblut; 100µl, 3-Gurke; 50 mg, 4-Tomatenpflanzenblatt; 100 mg; 5-Speichelprobe; 100µl, 6-Geflügelleber; Lebensmittel gefroren; 5mg, 7-Geflügelleber; Lebensmittel gefroren; 20mg, 8-Haarwurzel, 9-Putensalami; 50mg, 10-Eibe, Nadeln, 100mg Alle Proben wurden in 500µl Lysepuffer (2% CTAB; 2% Polyvinylpyrolidon, 10mM Tris-HCl; 20mM EDTA und 1, 5M Ammoniumchlorid ) und mit Ausnahme aller pflanzlichen Proben unter Zugabe von 20µl Proteinase K (20mg/ml) bei 65 inkubiert.
Die Lysate wurden nachfolgend mit 200µl Isopropanol versetzt und auf eine Zentrifugationssäule mit einer Glasfasermembran (Micro Spin Säule; Fa. LIDA) überführt.
Zentrifugation für 2 min bei 12.000 rpm. Verwerfen des Filtrates und 2 maliges Waschen der Membran mit einem Waschpuffer (50 mM NaCl; 10 mM Tris HCl; 1 mM EDTA; 70% v/v Ethanol). Nach Ethanolentfemung durch einen kurzen Zentrifugationschritt (12.000 rpm für 2min) Zugabe von 50-200µl eines Elutionspuffers (10 mM Tris-HCl; pH 8,7) und Elution der DNA durch Zentrifugation für 1 min bei 10.000 rpm.
Jeweils 1/5 der eluierten DNA wurden auf ein Agarosegel geladen und nach Ethidiumbromidfärbung dargestellt (Abbildung 2).

### 3. Isolierung genomischer DNA aus Tupferproben aus Abstrichen der Mundschleimhaut

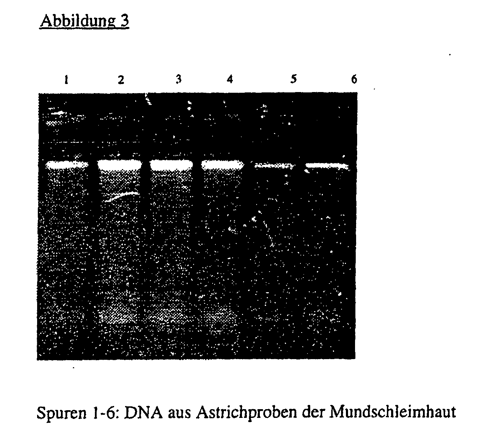

Die Isolierung der DNA aus Tupferproben von Abstrichen der Mundschleimhaut ist nachfolgend beschrieben.
Jeweils 400 µl Lyepuffer (CTAB, Polyvinylpyrolidone, Ammoniumchlorid, Tris, EDTA) wurde in ein 1.5 ml Eppendorf-Reaktionsgefüß überführt. In diesen Lysepuffer wurde der Tupfer mit dem Abstrichmaterial ausgedrückt und der Suspension 20 µl Proteinase K (20 mg/ml) zugegeben. Nachfolgend wurde der Ansatz bei 70°C für 10 min inkubiert
Nach Lyse wurden 200 µl eines Detergenz/Isopropanol-Gemisches zugegeben, die Probe kurz geschüttelt, nachfolgend auf eine kommerziell verfügbare Zentrifugationssäule (Fa. LIDA; Glasfasermembran) überführt und für 1 min bei 12.000 rpm zentrifugiert.
Die Säule wurde dann zweimal mit einem ethanolhaltigen Waschpuffer (NaCl, Tris-HCl; EDTA, Ethanol) gewaschen (zentrifugation bei 12.000 rpm; 1 min) und die Membran durch einen kurzen Zentrifugationsschritt getrocknet. Durch Zugabe von 200µl Elutionspuffer
(10 mM Tris-HCI) wurde die gebundene DNA von der Filtermembran durch einen kurzen Zentrifugationsschritt (10,000 rpm; 1min) eluiert.
Jeweils 20 µl der isolierten DNA aus beiden Extraktionsverfahren wurde zur Analyse auf ein 0.7% TAE-Agarosegel geladen und nach Ethidiumbromidfärbung analysiert (Abbildung 3).

### 4. Vergleich der erfindungsgemäßen DNA Extraktion aus Vollblutproben (200 µl) mit einem komerziellen Kit auf der Basis der Bindung der Nukleinsäuren unter Anwesenheit chaotroper Salze

Verglichen wurde die Isolierung genomischer DNA mittels des erfindungsgemäßen Verfahrens mit einem kommerziell verfügbaren und herkömmlich genutzten Verfahren zur Isolierung genomischer DNA unter Nutzung chaotroper Salze zur Nukleinsäurenbindung.
Die Extraktion genomischer DNA mittels des Vergleichsverfahrens erfolgte anhand der Anwendungsvorschrift.

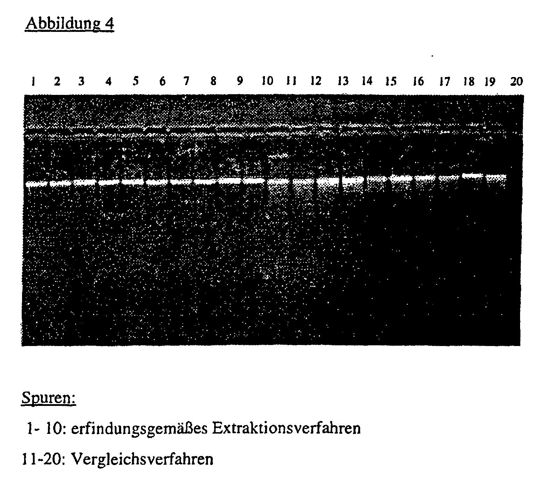

Die Isolierung der DNA mittels des erfindungsgemäßen Verfahrens ist nachfolgend beschrieben.
Jeweils 200 µl einer Vollblutprobe (EDTA behandelt; frisch) wurde in ein 1.5 ml Eppendorf-Reaktionsgefäß überführt.
Nach Zugabe von 350 µl eines Lysepuffers (CTAB, Polyvinylpyrolidone, Ammoniumchlorid, Tris, EDTA) und 20 µl Proteinase k (20 mg/ml) erfolgte eine Inkubation bei 70°C für 10 min zur Lyse des Ausgangsmaterials.
Nach Lyse wurden 180 µl eines Detergenz/Isopropanol-Gemisches zugegeben, die Probe kurz geschüttelt, nachfolgend auf eine kommerziell verfügbare Zentrifugationssäule (Fa. LIDA; Glasfasermembran) überführt und für 2 min bei 12.000 rpm zentrifugiert.
Die Säule wurde dann zweimal mit einem ethanolhaltigen Waschpuffer (NaCl, Tris-HCI; EDTA, Ethanol) gewaschen (zentrifugation bei 12.000 rpm; 1 min) und die Membran durch einen kurzen Zentrifugationsschritt getrocknet Durch Zugabe von 200µl Elutionspuffer (10 mM Tris-HCl) wurde die gebundene DNA von der Filtermembran durch einen kurzen Zentrifugationsschritt ( 10,000 rpm; 1min) eluiert.
Jeweils 10 µl der isolierten DNA aus beiden Extraktionsverfahren wurde zur Analyse auf ein 0.7% TAE-Agarosegel geladen und nach Ethidiumbromidfärbung analysiert.
Verglichen wurde die Ausbeuten an genomischer DNA, deren Integrität (saubere Einzelbande ohne niedermoleulare Schmierbanden) und die Reproduzierbarkeit der Extraktionsvefahren. Wie zu ersehen können mittels des erfindungsgemäßen Verfahrens bessere Ergebnisse als mittels des Vergleichsverfahrens erreicht werden (Abbildung 4).

### 5. Vergleich der erfindungsgemäßen DNA Extraktion aus Vollblutproben (5 µl) mit einem komerziellen Kit auf der Basis der Bindung der Nukleinsäuren unter Anwesenheit chaotroper Salze

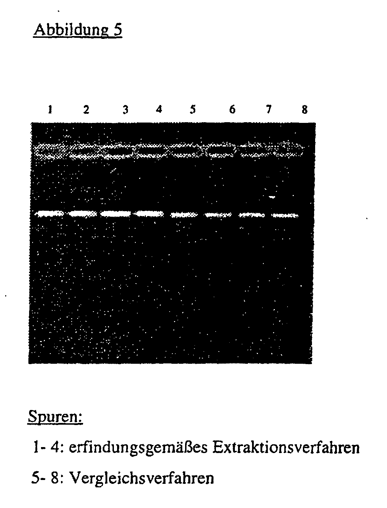

Verglichen wurde die Isolierung genomischer DNA mittels des erfindungsgemäßen Verfahrens mit einem kommerziell verfügbaren und herkömmlich genutzten Verfahren zur Isolierung genomischer DNA unter Nutzung chaotroper Salze zur Nukleinsäurenbindung.
Die Extraktion genomischer DNA mittels des Vergleichsverfahrens erfolgte anhand der Anwendungsvorschrift.
Die Isolierung der DNA mittels des erfindungsgemäßen Verfahrens ist nachfolgend beschrieben.
Jeweils 5 µl einer Vollblutprobe (EDTA behandelt; frisch) wurde in ein 1.5 ml Eppendorf-Reaktionsgefäß überführt.
Die Probe wurde durch Zuabe von 195 µl 1xPBS Puffer auf ein Volumen von 200 µl aufgefüllt und nach Zugabe von 350 µl eines Lysepuffers (CTAB, Polyvinylpyrolidone, Ammoniumchlorid, Tris, EDTA) und 20 µl Proteinase K (20mg/ml) erfolgte eine Inkubation bei 70°C für 10 min zur Lyse des Ausgangsmaterials.
Nach Lyse wurden 180 µl eines Detergenz/Isopropanol-Gemisches zugegeben, die Probe kurz geschüttelt, nachfolgend auf eine kommerziell verfügbare Zentrifugationssäule (Fa. LIDA; Glasfasermembran) überführt und für 2 min bei 12.000 rpm zentrifugiert.
Die Säule wurde dann zweimal mit einem ethanolhaltigen Waschpuffer (NaCl, Tris-HCI; EDTA, Ethanol) gewaschen (Zentrifugation bei 12.000 rpm; 1 min) und die Membran durch einen kurzen Zentrifugationsschritt getrocknet. Durch Zugabe von 200µl Elutionspuffer (10 mM Tris-HCl) wurde die gebundene DNA von der Filtermembran durch einen kurzen Zentrifugationsschritt ( 10,000 rpm; 1 min) eluiert.
Jeweils 20 µl der isolierten DNA aus beiden Extraktionsverfahren wurde zur Analyse auf ein 0.7% TAE-Agarosegel geladen und nach Ethidiumbromidfärbung analysiert.
Nachgewiesen und verglichen wurde die Möglichkeit der Isolierung genomischer DNA aus sehr geringen Mengen an Ausgangsmaterial und die Reproduzierbarkeit der Extraktionsvefahren. Wie zu ersehen können mittels des erfindungsgemäßen Verfahrens bessere Ergebnisse als mittels des Vergleichsverfahrens erreicht werden (Abbildung 5).

### 6.Vergleich der erfindungsgemäßen DNA Extraktion aus unterschiedlichen Arten tierischer Gewebeproben und unterschiedlicher Mengen an Ausgangsmaterial mit einem kommerziellen Kit auf der Basis der Bindung der Nukleinsäuren unter Anwesenheit chaotroper Salze

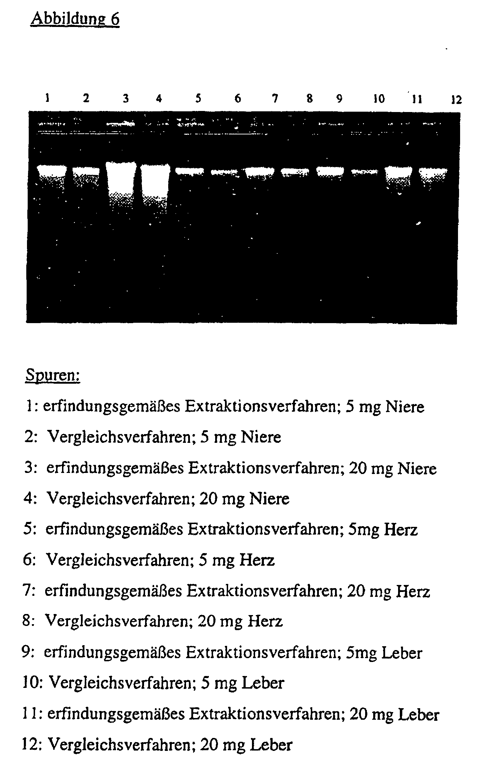

Verglichen wurde die Isolierung genomischer DNA mittels des erfindungsgemäßen Verfahrens mit einem kommerziell verfügbaren und herkömmlich genutzten Verfahren zur Isolierung genomischer DNA unter Nutzung chaotroper Salze zur Nukleinsäurenbindung.
Die Extraktion genomischer DNA mittels des Vergleichsverfahrens erfolgte anhand der Anwendungsvorschrift.
Die Isolierung der DNA mittels des erfindungsgemäßen Verfahrens ist nachfolgend beschrieben.
Jeweils 5 mg bzw. 20 mg von Gewebeproben aus Schweineniere, Schweineherz und Schweineleber wurden in ein 1.5 ml Eppendorf-Reaktionsgefäß überführt.
Der Probe wurden 400 µl eines Lysepuffers (CTAB, Polyvinylpyrolidone, Ammoniumchlorid, Tris, EDTA) und 40 µl Proteinase K (20 mg/ml) zugegeben.
Die Lyse des Ausgangsmaterials erfolgte durch Inkubation bei 52°C .
Nach der Lyse wurden durch einen kurzen Zentrifgationschritt (14.000 rpm; 1 min) eventuell nicht lysierte Bestandteile abzentrifugiert und der Überstand in einem neuen Reaktionsgefäß mit 200 µl eines Detergenz/Isopropanol-Gemisches zugegeben, die Probe kurz geschüttelt, nachfolgend auf eine kommerziell verfügbare Zentrifugationssäule (Fa. LIDA; Glasfasermembran) überführt und für 2 min bei 12.000 rpm zentrifugiert.
Die Säule wurde dann zweimal mit einem ethanolhaltigen Waschpuffer (NaCl, Tris-HCl; EDTA, Ethanol) gewaschen (Zentrifugation bei 12.000 rpm; 1 min) und die Membran durch einen kurzen Zentrifugationsschritt getrocknet. Durch Zugabe von 200µl Elutionspuffer (10 mM Tris-HCl) wurde die gebundene DNA von der Filtermembran durch einen kurzen Zentrifugationsschritt ( 10,000 rpm; 1min) eluiert.
Jeweils 10 µl der isolierten DNA aus beiden Extraktionsverfahren wurde zur Analyse auf ein 0.7% TAE-Agarosegel geladen und nach Ethidiumbromidfärbung analysiert.
Nachgewiesen und verglichen wurde die Möglichkeit der Isolierung genomischer DNA aus verschiedenen Gewebeproben sowie verschiedener mengen an Ausgangsmaterial hinsichtlich der Ausbeuten an genomischer DNA, deren Integrität (saubere Einzelbande ohne niedermoleulare Schmierbanden) und der Reproduzierbarkeit der Extraktionen.
Wie zu ersehen können mittels des erfindungsgemäßen Verfahrens bessere Ergebnisse als mittels des Vergleichsverfahrens erreicht werden (Abbildung 6).

### 7. DNA Extraktion aus Vollblutproben (200 µl) mittels des erfindungsgemäßen Verfahrens und Bindung der Nukleinsäuren an verschiedene für die Bindung unter Vermittlung chaotroper Salze eingesetzter Trägermaterialien

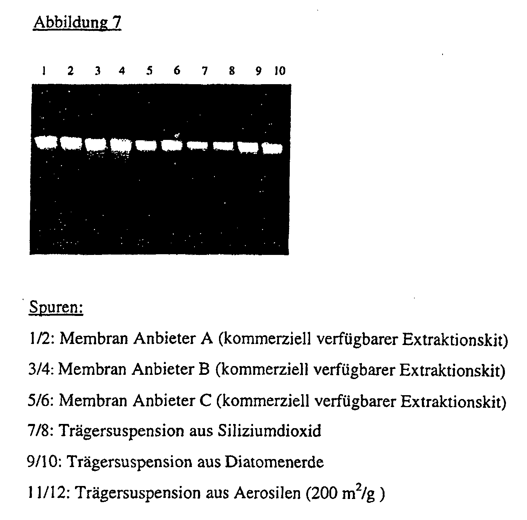

Dargestellt wird die Isolierung genomischer DNA mittels des erfindungsgemäßen Verfahrens aus 200 µl Vollblut und die Bindung der Nukleinsäuren an für die Isolierung von Nukleinsäuren mittels chaotroper Agentien Verwendung findenden verschiedenen Trägermaterialien (Säulenmembranen und Suspensionen).
Die Extraktion der DNA erfolgte wie in Ausführungsbeispiel 4 beschrieben, wobei anstelle der Glasfasemembran der Fa. LIDA verschiedene weitere Trägermaterialien eingesetzt wurden.
Jeweils 20 µl der isolierten DNA wurde zur Analyse auf ein 0.7% TAE-Agarosegel geladen und nach Ethidiumbromidfärbung analysiert.
Wie in der Abbildung 7 zu ersehen, realisiert das erfindungsgemäße Verfahren die Bindung der Nukleinsäuren an für die bisher bekannten chaotropen Verfahren eingesetzten unterschiedlichen Trägermaterialien.

### 8. Herstellung eines lagerstabilen Lysepuffersystems einschließlich eines proteolytischen Enzyms (Puffermix 1) und Verwendung des Lysepuffersystems für die Isolierung genomischer DNA aus verschiedenen Ausgangsmaterialien

Herstellung einer Lysepufferstammlösung enthaltend 3M Kaliumchlorid, 2% CTAB; 18,2 mM Tris-HCl (pH 8.3), 12,5 mM EDTA; 2,8% Polyvinylpyrrolidone. Aliquotieren von jeweils 400 µl der Stammlösung in 1.5 ml Eppendorf-Reaktionsgefäße und Zugabe von 40µl Proteinase K (20mg/ml).
Lyophilisation der Lysepuffermixturen in einer Lyophilisationsanlage (Alpha 2; Fa. Christ).
Nachfolgende Lagerung der Lysepuffermixturen in verschlossenen Reaktionsgefäßen bei Raumtemperatur für 6 Monate.

Die Extraktion der genomischen DNA erfolgte aus:
A: 500µl Vollblut
B: 400 µl Speichelprobe
C: Deparaffiniertem Gewebematerial.

### 1. DNA Extraktion aus Vollblut

Zugabe der 500 µl Vollblut auf die feste Formulierung des Lysepuffers und Inkubation bei 70°C für 10 min. Zugabe von 200µl Isopropanol und Überführung der Suspension auf eine Zentrifugationssäule (Glasfaserflies).
Zentrifugation für 2 min bei Maximalgeschwindigkeit und verwerfen des Zentrifugates.
Zugabe von 600 µl eies Waschpuffers (70% Ethanol, NaCl, Tris, EDTA), Zentrifugation für 1 min bei Maximalgeschwindigkeit und verwerfen des Zentrifugates. Wiederholung des Waschschrittes. Nachfolgend Trocknung der Membran durch Zentrifugation für 2 min bei Maximalgeschwindigkeit.
Elution der DNA von der Membran durch Zugabe von 200 µl eines Elutionspuffers (70°C) und Zentrifugation für 1 min bei Maximalgeschwindigkeit.

### 2. DNA Extraktion aus Speichelproben

Zugabe der 500 µl Speichelprobe auf die feste Formulierung des Lysepuffers und Inkubation bei 70°C für 10 min.Zugabe von 200µl Isopropanol und Überführung der Suspension auf eine Zentrifugationssäule (Glasfaserflies).
Zentrifugation für 2 min bei Maximalgeschwindigkeit und Verwerfen des Zentrifugates. Zugabe von 600 µl eines Waschpuffers (70% Ethanol, NaCl, Tris, EDTA), Zentrifugation für 1 min bei Maximalgeschwindigkeit und Verwerfen des Zentrifugates. Wiederholung des Waschschrittes. Nachfolgend Trocknung der Membran durch Zentrifugation für 2 min bei Maximalgeschwindigkeit.
Elution der DNA von der Membran durch Zugabe von 200 µl eines Elutionspuffers (70°C) und Zentrifugation für 1 min bei Maximalgeschwindigkeit.

### 3. DNA Extraktion aus deparaffiniertem Gewebe

Zugabe des deparaffiniertem Gewebestückchens auf die feste Formulierung des Lysepuffers, Zugabe von 500 µl ddH₂O und Inkubation bei 52°C für 30 min.
Zugabe von 200µl Isopropanol und Überführung der Suspension auf eine Zentrifugationssäule (Glasfaserflies).
Zentrifugation für 2 min bei Maximalgeschwindigkeit und Verwerfen des Zentrifugates.
Zugabe von 600 µl eies Waschpuffers (70% Ethanol, NaCl, Tris, EDTA), Zentrifugation für 1 min bei Maximalgeschwindigkeit und Verwerfen des Zentrifugates. Wiederholung des Waschschrittes. Nachfolgend Trocknung der Membran durch Zentrifugation für 2 min bei Maximalgeschwindigkeit.
Elution der DNA von der Membran durch Zugabe von 200 µl eines Elutionspuffers (70°C) und Zentrifugation für 1 min bei Maximalgeschwindigkeit.

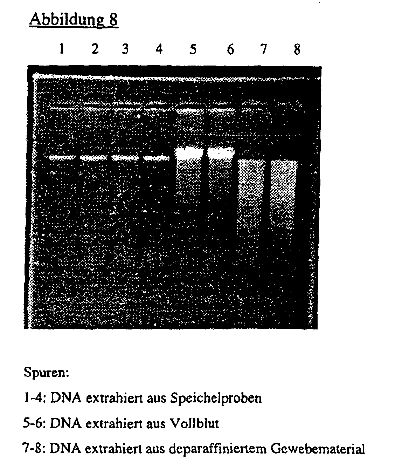

Die extrahierte DNA wurde anschließend gelelektrophoretisch analysiert.
Dazu wurden jeweils 1/10 des Gesamteluates an DNA aufgetragen (Abb. 8).

### 9. Herstellung eines lagerstabilen Lysepuffersystems einschließlich Proteinase K (Puffermix 2) und Verwendung des Lysepuffersystems für die Isolierung genomischer DNA aus 8 individuellen 100 µl Vollblutproben

Herstellung einer Lysepufferstammlösung enthaltend 3 M Ammoniumchlorid; 2% Polyvinylpyrolidone; 16,7 mM EDTA; 60 mM Tris-HCI; 1,6 % CTAB; 20 µl Proteinase K-(20 mg/ml).
Aliquotieren von jeweils 400 µl der Stammlösung in 1.5 ml Eppendorf-Reaktionsgefäße und Inkubation der geöffneten Eppendorf-Reaktionsgefäße in einem Thermomixer bei 95°C bis zur vollständigen Eintrocknung. Nachfolgend Verschließen der Reaktionsgefäße und Lagerung für 12 Monate bei Raumtemperatur.

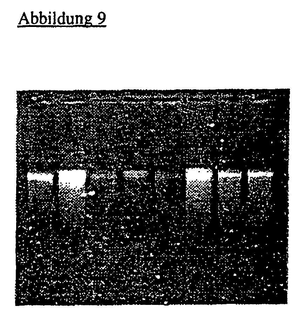

DNA Extraktion aus Vollblut
Zugabe der 100 µl Vollblut auf die feste Formulierung des Lysepuffers und Inkubation bei 70°C für 10 min. Zugabe von 20 µl einer mineralischen Carrier Suspension auf Silicabasis und kurzes Mixen. Inkubation des Ansatzes für 1 min. Pelletierung des Trägermaterials durch kurzes Anzentrifugieren. Waschen des Trägerpellets mit 800 µl eines Waschpuffers (70% Ethanol, NaCl, Tris, EDTA) und nachfolgende Entfernung des restlichen Ethanols durch Inkubation bei 70°C. Elution der DNA vom Trägermaterial durch Zugabe von 200 µl eines auf 70°C erwärmten Elutionspuffers (10 mM Tris-HCI; pH 8,69 und Abtrennung der Nukleinsäure vom Trägermaterial durch 1 minütige Zentrifugation bei Maximalgeschwindigkeit für 1min sowie Überführung der Nukleinsäure in ein neues Reaktionsgefäß.Die extrahierte DNA wurde anschließend gelelektrophoretisch analysiert.
Dazu wurden jeweils 1/10 des Gesamteluates an DNA aufgetragen (Abb. 9).

### 10. Isolierung genomischer DNA aus peripheren Blutlymphocyten durch direkte Bindung an funktionalisierte Oberflächen einer Mikrotestplatte

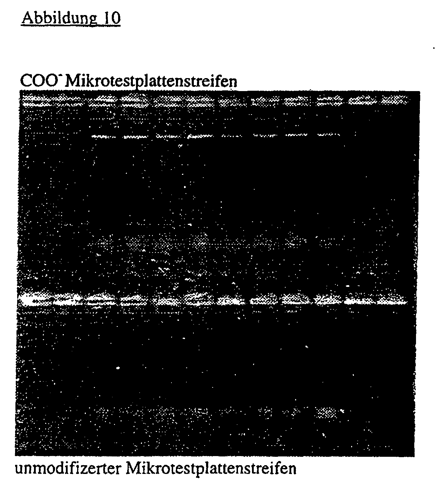

Als Mikrotestplatte wurde eine kommerziell verfügbare Platte mit einer COO⁻ Gruppenbelegung verwendet.
Jeweils ein Streifen der Platte (8-Wells) mit funktionellen Gruppen und eine Streifen ohne COO⁻Gruppenn als Negativkontrolle wurde für die Isolierung verwendet.
Alle Wells wurde mit 30 µl peripherer Blutlymphocyten in 1 x PBS Puffer beladen und mit 180µl eines Lysepuffers (Ammoniumchlorid; CTAB; Polivinylpyrolidone, Tris-HCl; EDTA; Proteinase K) versetzt und bei 70°C für 5 min inkubiert. Danach erfolgte die Zugabe von 80 µl eines Detergenz/Isopropanol-Gemisches. Die Ansätze wurden kurz geschüttelt und für 5 min inkubiert. Anschließend wurde die Lösungen aus den Wells abgegossen. Jedes Well wurde nachfolgend 2 x mit einem ethanolhaltigen Waschpuffer gespült und der restliche Ethanol durch kurze Inkubation bei 70°C entfernt.
Die Elution der Nukleinsäuren erfolgte durch Zugabe von 25 µl 10mM Tris-HCl und einer Inkubation für 2 min.
Die Eluate wurde anschließend auf einem 0.7% Aggarosegel ausgewertet. (Abbildung 10)

## Patentansprüche

1. Formulierungen ohne chaotrope Bestandteile zur Isolierung von Nukleinsäuren unter Bindung an eine feste Phase, insbesondere von DNA aus beliebigen komplexen Ausgangsmaterialien enthaltend
- ein Lyse/Bindungspuffersystem, das mindestens eine antichaotrope Salzkomponente aufweist,
- eine feste Phase,
- an sich bekannte Wasch- und Elutionspuffer.

2. Formulierungen nach Anspruch 1, **dadurch gekennzeichnet, daß**
die antichaotrope Komponente ein Ammonium-, Cäsium-, Natrium- und/oder Kaliumsalz ist, vorzugsweise Ammoniumchlorid.

3. Formulierungen nach Anspruch 1 oder 2, **dadurch gekennzeichnet, daß**
das Lyse/Bindungspuffersystem Detergenzien und ggf. Zusatzstoffe aufweist.

4. Formulierungen nach Anspruch 3, **dadurch gekennzeichnet, daß**
Detergenzien und Zusatzstoffe Tris-HCI, EDTA, Polyvinylpyrrolidone, CTAB, TritonX-100, N-Lauryl-Sarkosin, Natriumcitrat, DTT, SDS und/oder Tween sind.

5. Formulierungen nach einem der Ansprüche 1 bis 4, **dadurch gekennzeichnet, daß**
das Lyse/Bindungspuffersystem zur Bindung an die feste Phase einen Alkohol aufweist.

6. Formulierungen nach einem der Ansprüche 1 bis 5, **dadurch gekennzeichnet, daß**
das Lyse/Bindungspuffersystem Enzyme, vorzugsweise Protein-abbauende Enzyme, aufweist.

7. Formulierungen nach einem der Ansprüche 1 bis 6, **dadurch gekennzeichnet, daß**
das Lyse/Bindungspuffersystem als wässerige Lösung vorliegt.

8. Formulierungen nach einem der Ansprüche 1 bis 6, **dadurch gekennzeichnet, daß**
das Lyse/Bindungspuffersystem als feste lagerstabile Formulierung in einsatzfertigen Reaktionsgefäßen vorliegt.

9. Formulierungen nach einem der Ansprüche 1 bis 8, **dadurch gekennzeichnet, daß**
als feste Phase alle Trägermaterialien fungieren, die zur Isolierung mittels chaotroper Reagentien Anwendung finden, vorzugsweise Glafaservliese, Glasmembranen, Gläser, Zeolithe, Keramik, Siliciumträger.

10. Formulierungen nach einem der Ansprüche 1 bis 8, **dadurch gekennzeichnet, daß**
als feste Phase Trägermaterialien fungieren, die eine negativ funktionalisierte Oberfläche besitzen oder funktionalisierte Oberflächen aufweisen, die in ein negatives Ladungspotential überführt werden können.

11. Formulierungen nach Anspruch 10, **dadurch gekennzeichnet, daß**
die Oberfläche des Trägermaterials mit einer Acetylgruppe, Carboxylgruppe oder Hydroxylgruppe modifiziert ist.

12. Verfahren zur Isolierung von Nukleinsäuren, insbesondere von DNA, aus beliebigen komplexen Ausgangsmaterialien unter Verwendung von Formulierungen nach einem der Ansprüche 1 bis 9, **dadurch gekennzeichnet, daß**
das Ausgangsmaterial lysiert wird,
die Bindung der Nukleinsäuren an eine feste Phase erfolgt,
die am Träger gebundenen Nukleinsäuren gewaschen werden und die
Elution der Nukleinsäuren erfolgt.

13. Verfahren zur Isolierung von Nukleinsäuren nach Anspruch 12, **dadurch gekennzeichnet, daß** man das DNA enthaltende Material mit einem
- Lyse/Bindungspuffersystem, umfassend eine wässerige Lösung, die eine antichaotrope Salzkomponente, mindestens ein Detergenz, ggf. Zusatzstoffe sowie ggf. ein proteolytischen Enzym enthält, und
- mit einer festen Phase ggf. unter Zusatz eines Alkohols in Kontakt bringt,
- anschließend wäscht und die Nukleinsäure von der festen Phase löst.

14. Verfahren nach Anspruch 13, **dadurch gekennzeichnet, daß** Ausgangsmaterialien kompakte Pflanzenmaterialien, wie Früchte; Samen; Blätter; Nadeln etc., klinisch relevante Proben, wie Vollblut; Gewebe, Mikrobioptate, paraffinierte Materialien, ercp-proben, Tupfermaterial von Abstrichen, Lebensmittel, wie Fisch, Wurst, Konserven, Milch, forensischen Proben, wie Haarwurzeln, Zigarettenkippen, Blutspuren und andere Proben, die DNA enthalten, sind.

15. Verfahren nach einem der Ansprüche 12 bis 14, **dadurch gekennzeichnet, daß** die Ionenstärken der antichaotropen Salze zur Lyse/Bindung zwischen 0,1 und 8 M liegen.

16. Verfahren zur Isolierung von Nukleinsäuren, insbesondere von DNA, aus beliebigen komplexen Ausgangsmaterialien unter Verwendung von Formulierungen nach einem der Ansprüche 1 bis 8 und 10 bis 11, **dadurch gekennzeichnet, daß**
- das Ausgangsmaterial in einem "Single Tube"- bzw. Einschritt-Verfahren mit einer negativ funktionalisierten Oberfläche oder dessen Oberfläche chemisch so modifiziert ist, daß sie in ein negatives Ladungspotential überführt werden kann, in Kontakt gebracht und lysiert wird,
- die Bindung der Nukleinsäure an die Oberfläche erfolgt,
- die gebundene Nukleinsäure gewaschen und ggf. elutiert wird.

17. Verfahren nach Anspruch 16, **dadurch gekennzeichnet, daß**
negativ funktionalisierte Oberfächen entsprechend modifizierte planare Oberflächen, Filtermembranen, herrkömmliche Plastikgefäße oder Mikrotestplatten sind.

18. Verfahren nach Anspruch 16 oder 17, **dadurch gekennzeichnet, daß** die Nukleinsäure anschließend im gleichen Reaktionsansatz einer Amplifikationsreaktion ausgewählter Sequenzabschnitte unterzogen und ggf. daran anschließend eine Analyse der Gensequenzen durchgeführt wird.

19. Verfahren nach Anspruch 16 oder 17, **dadurch gekennzeichnet, daß** die Nukleinsäure anschließend im gleichen Reaktionsansatz hybridisiert oder sequenziert wird.

20. Verwendung antichaotroper Komponenten in einem Lyse/Bindungspuffersystem zur Isolierung und Reinigung von Nukleinsäuren unter Bindung an eine feste Phase.

21. Verwendung nach Anspruch 20, **dadurch gekennzeichnet, daß** antichaotrope Komponenten Ammonium-, Cäsium-, Natrium- und/oder Kaliumsalze sind, vorzugsweise Ammoniumchlorid.

22. Verwendung nach Anspruch 20 oder 21, **dadurch gekennzeichnet, daß** zur Lyse/Bindung die antichaotrpen Salze in Ionenstärken von 0,1 bis zu 8 M eingesetzt werden.

23. Verwendung nach einem der Ansprüche 20 bis 22, dadurch gekenzeichnet, daß das Lyse/Bindungspuffersystem als wässerige Lösung eingesetzt wird.

24. Verwendung nach einem der Ansprüche 20 bis 22, dadurch gekenzeichnet, daß das Lyse/Bindungspuffersystem als feste lagerstabile Formulierung vorliegt.

25. Verwendung nach einem der Ansprüche 20 bis 24 zur präparativen Isolierung und Aufreinigung für DNA zum Einsatz in der Gentherapie.

## Claims

1. Formulations without chaotropic components for the isolation of nucleic acids with binding to a solid phase, in particular of DNA from arbitrary complex initial materials, comprising
- a lysis/binding buffer system, manifesting at least one anti-chaotropic salt component,
- one solid phase
- washing and elution buffers known per se.

2. Formulations according to Claim 1, wherein
the anti-chaotropic component is an ammonium, caesium, sodium and/or potassium salt, preferably ammonium chloride.

3. Formulations according to Claims 1 or 2, wherein
the lysis/binding buffer system manifests detergents and, if applicable, additives.

4. Formulations according to Claim 3, wherein
the detergents and additives are Tris-HCl, EDTA, polyvinyl pyrrolidone, CTAB, TritonX-100, N-lauryl-sarcosine, sodium citrate, DTT, SDS and/or Tween.

5. Formulations according to one of the Claims 1 to 4, wherein
the lysis/binding buffer system manifests an alcohol for binding onto the solid phase.

6. Formulations according to one of the Claims 1 to 5, wherein
the lysis/binding buffer system manifests enzymes, preferably protein-de-composing enzymes.

7. Formulations according to one of the Claims 1 to 6, wherein the lysis/buffer binding system is available as a watery solution.

8. Formulations according to one of the Claims 1 to 6, wherein the lysis/buffer binding system is available as a firm, storage-stable formulation in reaction vessels ready for use.

9. Formulations according to one of the Claims 1 to 8, wherein all carrier materials used for isolation by means of chaotropic reagents act as a solid phase, preferably fibre-glass fleeces, glass membranes, glasses, zeolites, ceramic, silicon carriers.

10. Formulations according to one of the Claims 1 to 8, wherein
carrier materials possessing a negatively functionalised surface or manifesting functionalised surfaces which can be converted into a negative charge potential act as carrier materials.

11. Formulations according to Claim 10, wherein
the surface of the carrier material has been modified with an acetyl group, carboxyl group or hydroxyl group.

12. Method for the isolation of nucleic acids, in particular of DNA, from arbitrary initial materials making use of formulations according to one of the Claims 1 to 9, wherein
the initial material is lysed,
the binding of the nucleic acid is done to a solid phase,
the nucleic acids bound to the carrier are washed and the
elution of the nucleic acids is carried out.

13. Method for the isolation of nucleic acids according to Claim 12, wherein the material containing DNA is brought into contact with a
- lysis/binding buffer system entailing a watery solution containing an anti-chaotropic salt component, at least one detergent, if need be additives, as well as, if need be, a proteolytic enzyme, and
- with a solid phase, if need be with addition of an alcohol,
- is subsequently washed and the nucleic acid dissolved from the solid phase.

14. Method according to Claim 13, wherein initial materials are compact vegetable materials such as fruits, seeds, leaves, needles etc., clinically relevant samples such as full blood, tissue, micro-bioptates, paraffin-coated materials, ercp samples, swab material from smear tests, foodstuffs such as fish, cooked meats, preserves, milk, forensic samples such as hair roots, cigarette ends, blood traces and other samples containing DNA.

15. Method according to one of the Claims 12 to 14, wherein the ion thickness of the anti-chaotropic salts for the lysis/binding is between 0.1 and 8 M.

16. Method for the isolation of nucleic acids, in particular of DNA, from arbitrary initial materials making use of formulations according to one of the Claims 1 to 8 and 10 to 11, wherein
- the initial material is brought into contact and lysed with a negatively functionalised surface in a "single tube" or single-step process or its surface is chemically modified in such a way that it can be converted into a negative charge potential,
- the binding of the nucleic acid is done to the surface
- the bound nucleic acid is washed and, if need be, eluted.

17. Method according to Claim 16, wherein
negatively functionalised surfaces are correspondingly modified planar surfaces, filter membranes, conventional plastic vessels or micro-test panels.

18. Method according to Claim 16 or 17, wherein the nucleic acid is subsequently subjected to an amplification reaction of selected sequence sections in the same reaction mixture and, if need be, an analysis of the gene sequences is subsequently implemented.

19. Method according to Claim 16 or 17, wherein the nucleic acid is subsequently hybridised or sequenced in the same reaction mixture.

20. Use of anti-chaotropic components in a lysis/binding buffer system for isolation and cleansing of nucleic acids with binding to a solid phase.

21. Use according to Claim 20, wherein anti-chaotropic components are ammonium, caesium, sodium and/or potassium salts, preferably ammonium chloride.

22. Use according to Claim 20 or 21, wherein the anti-chaotropic salts are used in thicknesses of 0.1 to 8 M for the lysis/binding.

23. Use according to one of the Claims 20 to 22, wherein the lysis/binding buffer system is used as a watery solution.

24. Use according to one of the Claims 20 to 22, wherein the lysis/buffer binding system is available as a firm, storage-stable formulation.

25. Use according to one of the Claims 20 to 24 for preparative isolation and cleansing for DNA for use in gene therapy.

## Revendications

1. Formulations sans éléments chaotropes pour isoler des acides nucléiques en les liant à une phase solide, en particulier l'ADN issu de n'importe quel matériau complexes de départ contenant
- un système tampon de lyse/liaison qui présente une composante salée antichaotrope,
- une phase solide,
- un tampon de lavage et d'élution connu en soi.

2. Formulations selon la revendication 1, se caractérisant par le fait que la composante antichaotrope est un sel d'ammonium, de césium, de sodium et/ou de potassium, de préférence un sel ammoniaque.

3. Formulations selon la revendication 1 ou 2, se caractérisant par le fait que le système tampon de lyse/liaison présente des détergents et le cas échéant des adjuvants.

4. Formulations selon la revendication 3, se caractérisant par le fait que les détergents et les adjuvants sont des tris-HCI, EDTA, polyvinylpyrrolidone, CTAB, TritonX-100, N-lauroyl-sarcosine, citrate de sodium, DTT, SDS et/ou Tween.

5. Formulations selon l'une des revendications 1 à 4, se caractérisant par le fait que le système tampon de lyse/liaison présente un alcool pour la liaison à la phase solide.

6. Formulations selon l'une des revendications 1 à 5, se caractérisant par le fait que le système tampon de lyse/liaison présente des enzymes, de préférence des enzymes décomposant des protéines.

7. Formulations selon l'une des revendications 1 à 6, se caractérisant par le fait que le système tampon de lyse/liaison se présente sous forme de solution aqueuse.

8. Formulations selon l'une des revendications 1 à 6, se caractérisant par le fait que le système tampon de lyse/liaison se présente sous forme de formulation solide, à position permanente dans des réacteurs prêts à l'emploi.

9. Formulations selon l'une des revendications 1 à 8, se caractérisant par le fait que tous les matériaux supports qui sont employés pour l'isolation au moyen de réactifs chaotropes, jouent le rôle de phase solide, matériaux supports de préférence tels que les non-tissus de coton de verre, les membranes en verre, les verres, les zéolithes, la céramique, les supports en silicium.

10. Formulations selon l'une des revendications 1 à 8, se caractérisant par le fait que les matériaux supports qui ont une surface fonctionnalisée négative ou présentent des surfaces fonctionnalisées qui peuvent être transformées en potentiel de charge négatif, jouent le rôle de phase solide.

11. Formulations selon la revendication 10, se caractérisant par le fait que la surface du matériau de support est modifié avec un groupe acétyle, un groupe carboxyle ou un groupe hydroxyle.

12. Procédé pour isoler les acides nucléiques, en particulier l'ADN, issu de n'importe quel matériau complexes de départ en employant les formulations selon l'une des revendications 1 à 9, se caractérisant par le fait
que le matériau de départ est lysé,
que la liaison des acides nucléiques se fait sur une phase solide,
que les acides nucléiques liés au support sont lavés et que l'élution des acides nucléiques est réalisée.

13. Procédé pour isoler les acides nucléiques selon la revendication 12, se caractérisant par le fait que le matériau contenant l'ADN est en contact avec un
- système tampon de lyse/liaison, englobant une solution aqueuse qui contient une composante salée antichaotrope, au moins un détergent, le cas échéant des adjuvants ainsi que le cas échéant une enzyme protéolytique, et
- en contact avec une phase solide, le cas échéant en y ajoutant un alcool,
- est ensuite lavé et l'acide nucléique détaché de la phase solide.

14. Procédé selon la revendication 13, se caractérisant par le fait que les matériaux de départ sont des matériaux végétaux compacts, tels que des fruits; des semences; des feuilles, des aiguilles etc., sont des prélèvements cliniques significatifs, tels que du sang entier; des tissus, des microproduits de biopsie, des matériaux paraffinés, des prélèvements ercp, sont des tampons de frottis, de denrées alimentaires, tels que du poisson, de la saucisse, des conserves, du lait, sont des prélèvements de médecine légale, tels que des racines de cheveux, des mégots de cigarettes, des traces de sang et autres prélèvements qui contiennent de l'ADN.

15. Procédé selon l'une des revendications 12 à 14, se caractérisant par le fait que les forces ioniques des sels antichaotropes pour la lyse/liaison se situent entre 0,1 et 8 M.

16. Procédé pour isoler les acides nucléiques, en particulier l'ADN, issu de n'importe quel matériau complexe de départ pour l'emploi de formulations selon les revendications 1 à 8 et 10 à 11, se caractérisant par le fait que
- le matériau de départ est mis en contact et lysé lors d'un procédé "Single Tube" ou procédé à un pas avec une surface fonctionnalisée négative ou dont la surface est modifiée de sorte qu'elle puisse être transformée en un potentiel de charge négatif,
- que la liaison de l'acide nucléique a lieu sur la surface,
- que l'acide nucléique lié est lavé et le cas échéant élué.

17. Procédé selon la revendication 16, se caractérisant par le fait que les surfaces fonctionnalisées négatives correspondant à des surfaces planaires modifiées sont des membranes filtrantes, des récipients en plastique usuels ou des plaques de microtest.

18. Procédé selon les revendications 16 ou 17, se caractérisant par le fait que l'acide nucléique sera soumis ensuite durant le même processus réactionnel à une réaction d'amplification de sections séquentielles choisies et que le cas échéant à la suite de ceci une analyse des séquences génétiques sera réalisée.

19. Procédé selon les revendications 16 ou 17, se caractérisant par le fait que l'acide nucléique est ensuite hybridé ou séquencé durant le même processus réactionnel.

20. Emploi de composante antichaotropes dans un système tampon de lyse/liaison pour isoler et nettoyer des acides nucléiques en les liant à une phase solide.

21. Emploi selon la revendication 20, se caractérisant par le fait que les composantes antichaotropes sont des sels d'ammonium, de césium, de sodium et/ou de potassium, de préférence du sel ammoniaque.

22. Emploi selon la revendication 20 ou 21, se caractérisant par le fait que les sels antichaotropes sont utilisés dans les forces ioniques de 0,1 à 8 M pour la lyse/liaison.

23. Emploi selon l'une des revendications 20 à 22, se caractérisant par le fait que le système tampon lyse/liaison est employé sous forme de solution aqueuse.

24. Emploi selon l'une des revendications20 à 22, se caractérisant par le fait que le système tampon de lyse/liaison se trouve sous forme de formulation solide, à position permanente.

25. Emploi selon l'une des revendications 20 à 24 pour l'isolation préparatoire et nettoyage pour ADN pour l'emploi dans la thérapie génique.
